(19) Europäisches Patentamt
European Patent Office
Office européen des brevets

(11) Publication number: **0 544 287 A1**

(12) **EUROPEAN PATENT APPLICATION**

(21) Application number: **92120196.8**

(22) Date of filing: **26.11.92**

(51) Int. Cl.5: **C07D 493/08**, A61K 31/41, A61K 31/557, //(C07D493/08, 307:00,307:00)

(30) Priority: **27.11.91 US 799233**

(43) Date of publication of application: **02.06.93 Bulletin 93/22**

(84) Designated Contracting States: **AT BE CH DE DK ES FR GB GR IE IT LI LU MC NL PT SE**

(71) Applicant: **E.R. SOUIBB & SONS, INC.**
**P.O.Box 4000**
**Princeton New Jersey 08543-4000(US)**

(72) Inventor: **Misra, Raj N.**
**12 Eaton Place**
**Hopewell, NJ(US)**

(74) Representative: **Vossius & Partner**
**Siebertstrasse 4 P.O. Box 86 07 67**
**W-8000 München 86 (DE)**

# (54) Gem-dialkyl-7-oxabicycloheptyl substituted heterocyclic amide prostaglandin analogs useful in the treatment of thrombotic and vasospastic disease.

(57) Gem-dialkyl-7-oxabicycloheptane substituted prostaglandin analogs useful in treating thrombotic and vasospastic disease have the structural formula

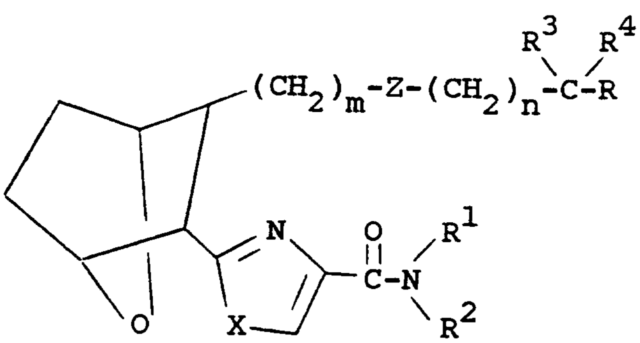

wherein m is 1, 2 or 3; n is 0, 1, 2, 3 or 4;
Z is $-(CH_2)_2-$, $-CH=CH-$ or

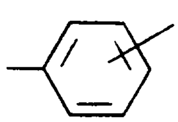

with the proviso when Z is $-CH=CH-$, n is l, 2, 3 or 4; R is $CO_2H$, $CO_2$lower alkyl, or $CO_2$alkali metal, X is O or NH; and where $R^1$ and $R^2$ are as defined herein and $R^3$ and $R^4$ are each independently alkyl, or $R^3$ and $R^4$ may

EP 0 544 287 A1

be taken together with the carbon to which it is attached to form a 3- or 4-membered ring.

The present invention relates to gem-dialkyl-7-oxabicycloheptyl substituted heterocyclic amide prostaglandin analogs which are thromboxane $A_2$ ($TXA_2$) receptor antagonists or combined thromboxane $A_2$ receptor antagonists/thromboxane synthetase inhibitors useful, for example, in the treatment of thrombotic and/or vasospastic disease. These compounds have the structural formula I

I

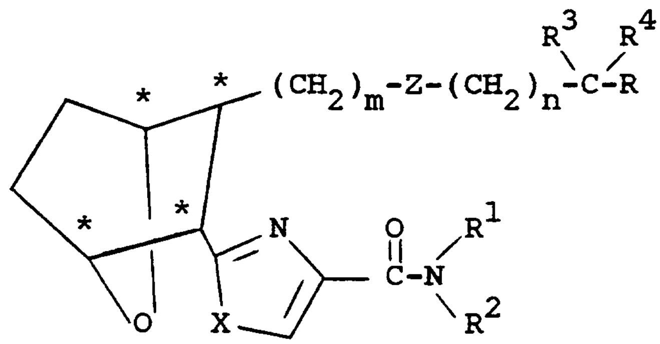

and including all stereoisomers thereof, wherein

m is 1, 2 or 3; n is 0, 1, 2, 3 or 4;

Z is $-(CH_2)_2-$, -CH = CH- or

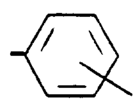 ,

with the proviso that when Z is -CH = CH-, n is l, 2, 3, or 4;

R is $CO_2H$, $CO_2$lower alkyl, or $CO_2$alkali metal;

X is O or NH;

$R^1$ is hydrogen, lower alkyl, lower alkenyl, lower alkynyl, aralkyl, aryl, cycloalkyl, cycloalkylalkyl, cycloheteroalkyl, cycloheteroalkylalkyl, heteroaryl or heteroarylalkyl, or amide

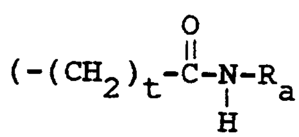 or 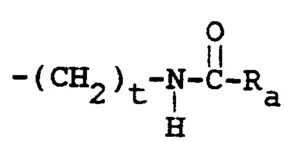

wherein t is l to l2 and $R_a$ is lower alkyl, aryl, cycloalkyl, or cycloalkylalkyl), each of $R^l$ being unsubstituted or optionally substituted with a lower alkyl, aryl, cycloalkyl, or cycloalkylalkyl group;

$R^2$ is hydrogen, lower alkyl, aryl, or aralkyl; or

$R^1$ and $R^2$ together with the nitrogen to which they are linked may form a 5- to 8- membered ring; and

$R^3$ and $R^4$ are the same or different and each is lower alkyl. $R^3$ and $R^4$ may be linked to form a 3- or 4-membered ring.

Thus, the compounds of the invention include the following types of compounds:

IA

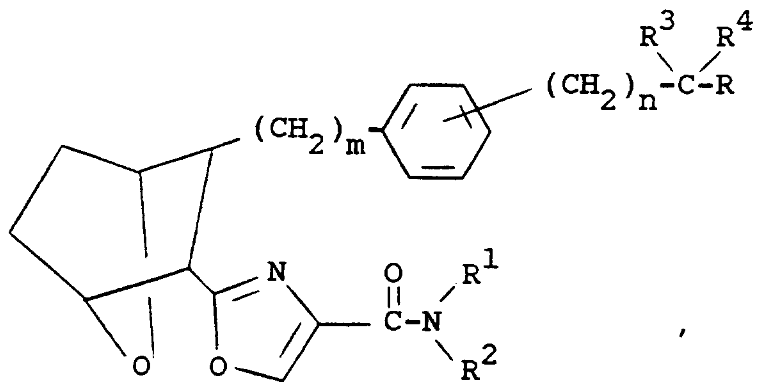

,

IB

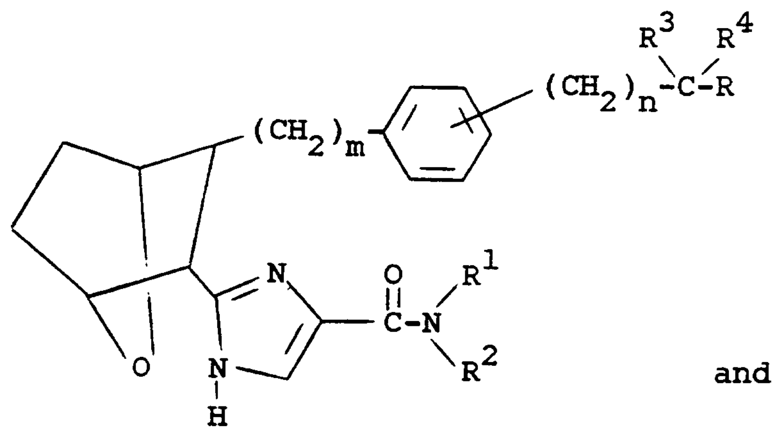

and

IC

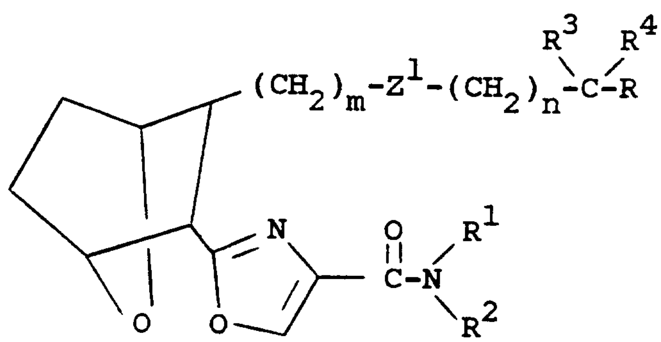

, and

ID

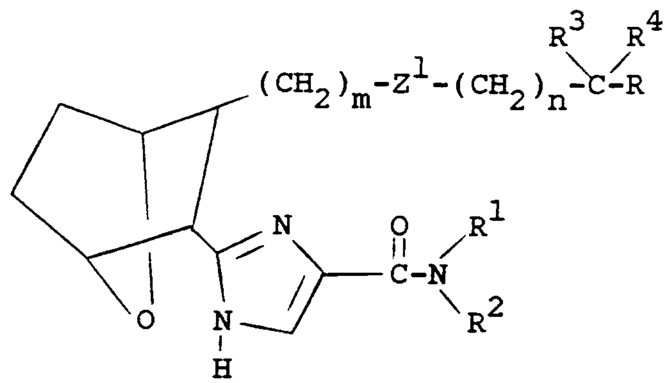

wherein in formulae IC and ID, $Z^l$ is -CH = CH- or $-(CH_2)_2-$.

The term "lower alkyl" or "alkyl" as employed herein includes both straight and branched chain radicals of up to 18 carbons, preferably 1 to l2 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, the various branched chain isomers thereof, and the like as well as such groups including 1, 2 or 3 halo substituents, an aryl substituent, an alkyl-aryl substituent, a haloaryl substituent, a cycloalkyl substituent, an alkylcycloalkyl substituent, hydroxy or a carboxy substituent.

The term "cycloalkyl" includes saturated cyclic hydrocarbon groups containing 3 to 12 carbons, preferably 3 to 8 carbons, which include cyclopropyl, cyclobutyl, cyclopentyl, cyclohexyl, cycloheptyl, cyclooctyl, cyclodecyl and cyclododecyl, any of which groups may be substituted with substituents such as halogen, lower alkyl, alkoxy and/or hydroxy group.

The term "aryl" or "Ar" as employed herein refers to monocyclic or bicyclic aromatic groups containing from 6 to l0 carbons in the ring portion, such as phenyl, naphthyl. Aryl (or Ar), phenyl or naphthyl may include substituted aryl, substituted phenyl or substituted naphthyl, which may include l or 2 substituents on either the phenyl or naphthyl such as lower alkyl, trifluoromethyl, halogen (Cl, Br, I or F), lower alkoxy, arylalkoxy, hydroxy, alkylthio, alkylsulfinyl, alkylsulfonyl, arylthio, arylsulfinyl and/or arylsulfonyl.

The term "aralkyl", "aryl-alkyl" or "aryl-lower alkyl" as used herein refers to lower alkyl groups as discussed above having an aryl substituent, such as benzyl.

The term "lower alkoxy", "alkoxy" or "aralkoxy" includes any of the above lower alkyl, alkyl or aralkyl groups linked to an oxygen atom.

The term "halogen" or "halo" as used herein refers to chlorine, bromine, fluorine or iodine with chlorine being preferred.

The term "lower alkenyl" or "alkenyl" as employed herein with respect to the $R^l$ substituent includes a carbon chain of up to l6 carbons, preferably 3 to l0 carbons, containing one double bond which will be separated from "N" by at least one saturated carbon moiety such as $-(CH_2)_q-$ where g can be l to l4, such as 2-propenyl, 2-butenyl, 3-butenyl, 2-pentenyl, 4-pentenyl and the like, and may include a halogen substituent such as I, Cl, or F.

The term "lower alkynyl" or "alkynyl" as employed herein with respect to the $R^l$ substituent includes a carbon chain of up to l6 carbons, preferably 3 to l0 carbons, containing one triple bond which will be separated from "N" by at least one saturated carbon moiety such as $-(CH_2)_{q'}-$ where g' can be l to l4, such as 2-propynyl, 2-butynyl, 3-butynyl and the like.

The term "cycloheteroalkyl" as used herein as an $R^l$ substituent refers to a 5-, 6- or 7-membered saturated ring which includes l or 2 hetero atoms such as nitrogen, oxygen and/or sulfur, and which is linked to the "N" of the

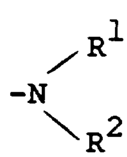

group through a carbon atom either beta or gamma to a heteroatom, such as

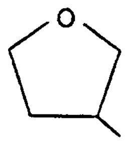 , 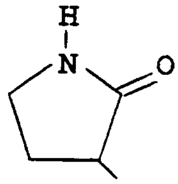 , 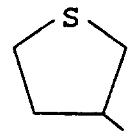 ,

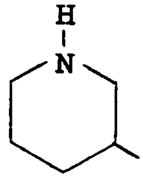 , 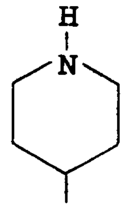

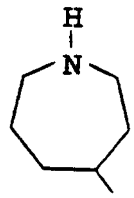 , 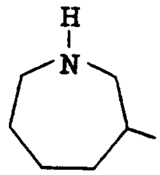

and the like.

The term "heteroaryl" or heteroaromatic as an $R^l$ substituent refers to a 5- or 6-membered aromatic ring which includes l or 2 hetero atoms such as nitrogen, oxygen or sulfur, which are not directly linked through a hetero
atom to the "N" of the

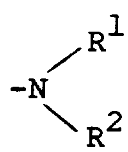

group,
such as

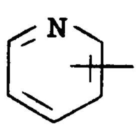 , 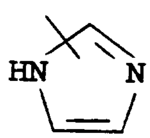 ,

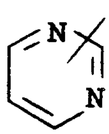 , 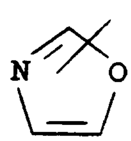 , 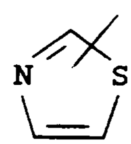

and the like

The term "cycloheteroalkylalkyl" as defined by $R^l$ refers to 5-, 6- or 7-membered saturated ring which includes l or 2 heteroatoms such as nitrogen, oxygen or sulfur, and is linked
to the "N" of the

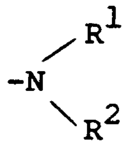

group through a $(CH_2)_x$ chain wherein x is l to l2, preferably l to 8, such as

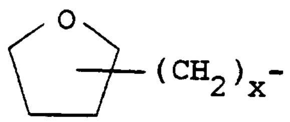 , 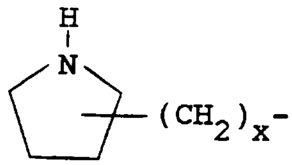 , 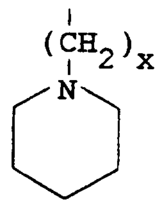 ,

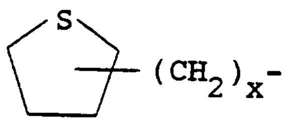 , 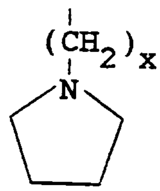 , 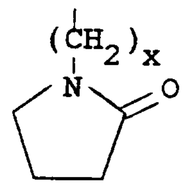

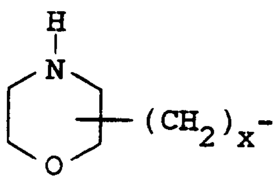 , 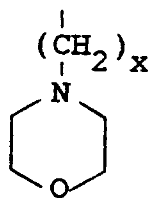 , 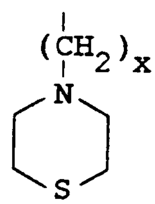 ,

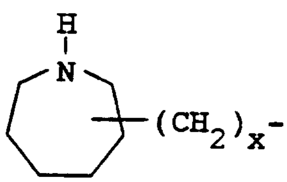 or 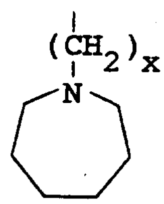

The term "heteroarylalkyl" as defined by $R^l$ refers to a 5-, 6- or 7-membered aromatic ring which includes l, 2, 3 or 4 heteroatoms such as nitrogen, oxygen or sulfur, and is linked to the "N" of the

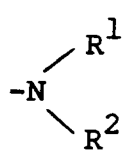

group through a $-(CH_2)_{x'}$-chain where x' is l to l2, preferably l to 8, such as

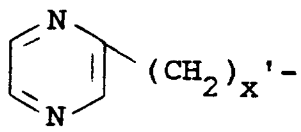 , 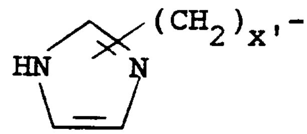 ,

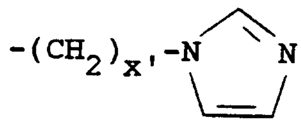 , 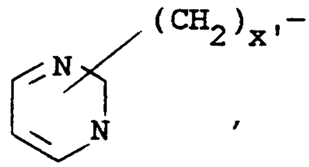 ,

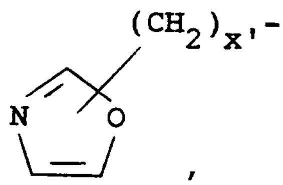 , 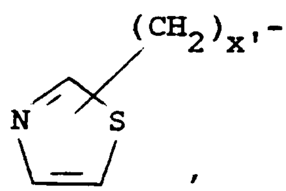 ,

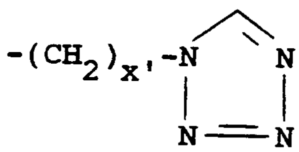 or 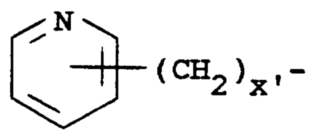

Preferred are those compounds of formula I wherein Z is

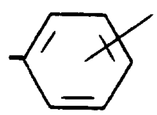

and X is O, and $R^3$ and $R^4$ are the same alkyl. More preferred are compounds of formula I wherein $R^3$ and $R^4$ are each methyl,
Z- is

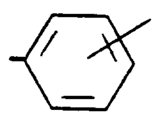 ,

m is l, n is l or 2,
X is O, R is $CO_2H$, $R^1$ is alkyl or substituted alkyl such as cycloalkylalkyl, such as cyclohexylbutyl and $R^2$ is H or lower alkyl such as methyl, and

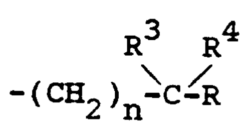

is in the ortho or meta position.

Also preferred are compounds of formula I wherein Z is -CH=CH- in the cis configuration, m is l, n is 2 or 3, R is $CO_2H$, $R^l$ is alkyl or substituted alkyl such as cycloalkylalkyl and $R^2$ is H or lower alkyl, such as

methyl, and $R^3$ and $R^4$ are each methyl.

The compounds of formula I of the invention may be prepared as follows.

The various compounds of the invention wherein Z is

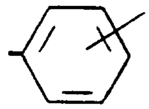

may be prepared as outlined below.

Compounds of the invention where X is O are prepared starting with bromophenylalkyl alcohol A

A

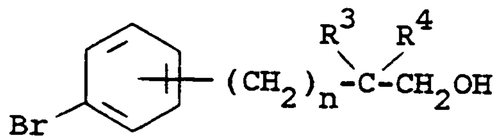

wherein n is 1, 2, 3 or 4 which is treated with a protecting compound such as dimethylthexylsilyl chloride, in the presence of an amine base such as triethylamine or 4-dimethylaminopyridine, and an inert solvent such as methylene chloride, employing conventional procedures, to form the protected bromophenylalkyl compound B

B

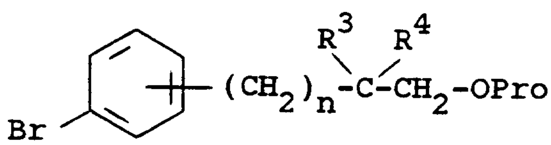

wherein Pro represents a protecting group.

Examples of protecting compounds suitable for use herein in reacting with bromophenalkyl alcohol A include but are not limited to

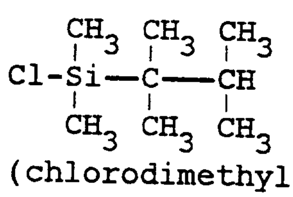

(chlorodimethylthexylsilane

or

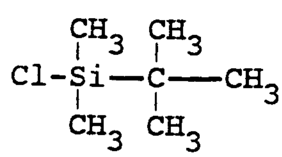

(chlorodimethyl-t-butylsilane)

The protected compound B is then converted to a Grignard reagent by treatment with magnesium in the presence of an inert organic solvent such as tetrahydrofuran (THF) or diethyl ether and then is condensed with (exo)octahydro-5,8-epoxy-IH-benzopyran-3-ol or (exo)octahydro-4,7-epoxyisobenzofuran-l-ol (prepared as described in U.S. Patent No. 4,143,054) of the structure C

C

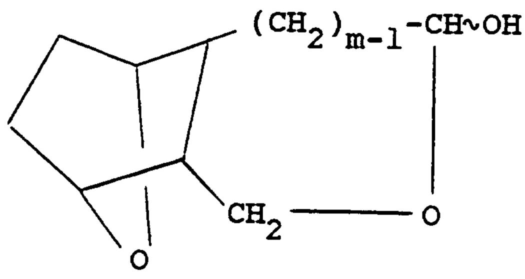

employing a molar ratio of C:B of within the range of from about 1:2 to about 1:4, in the presence of an inert organic solvent such as THF at a reduced temperature of from about -78 to about 25°C, to form the condensed 7-oxabicycloheptane compound II

II

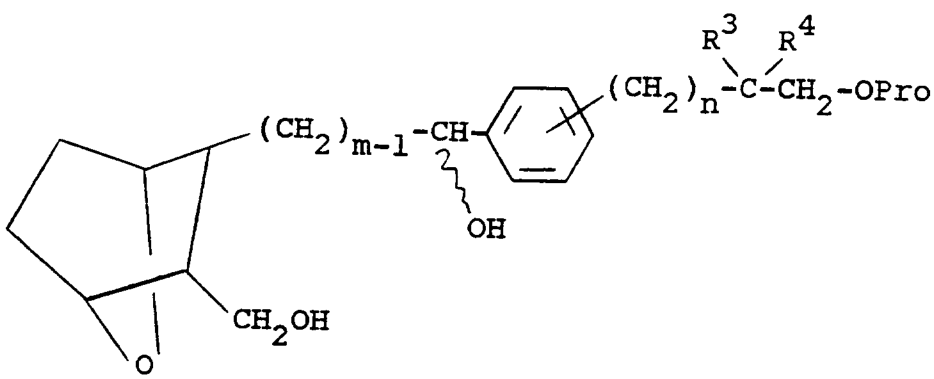

In a preferred method, compound II can be formed by treatment of C with ethylmagnesium bromide employing a molar ratio of C:ethylmagnesiun bromide of within the range of from about l:l to about l:0.9 in the presence of an inert organic solvent such as THF at a reduced temperature of from about -78° to about 0°C. Treatment of the resulting anion solution with the above Grighard reagent from compound B employing a molar ratio of C:Grignard B of within the range of l:l.l to l:l.5 at a temperature of about 0° to about 25°C forms compound II.

The condensed compound II is then subjected to hydrogenolysis by treatment with hydrogen in the presence of a catalyst such as palladium hydroxide on charcoal in acetic acid or an inert organic solvent such as methanol or ethyl acetate, to form the alcohol III

III

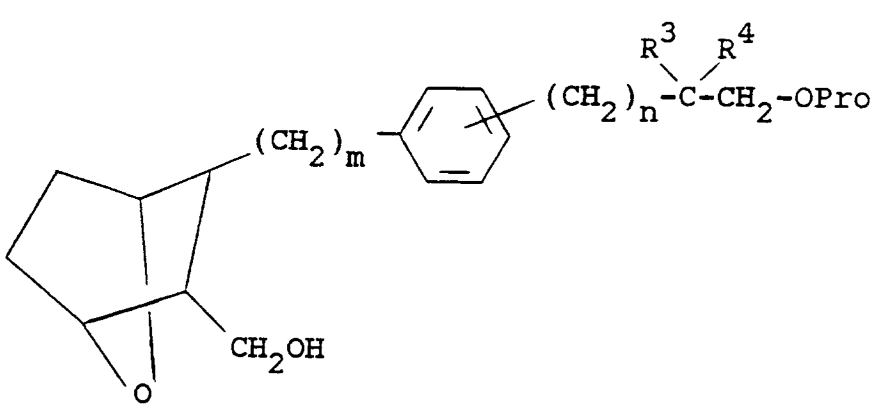

Alcohol III is subjected to acetylation by treatment with acetyl chloride or acetic anhydride in the presence of pyridine and methylene chloride to acetylate the free alcohol to form IIIA.

IIIA

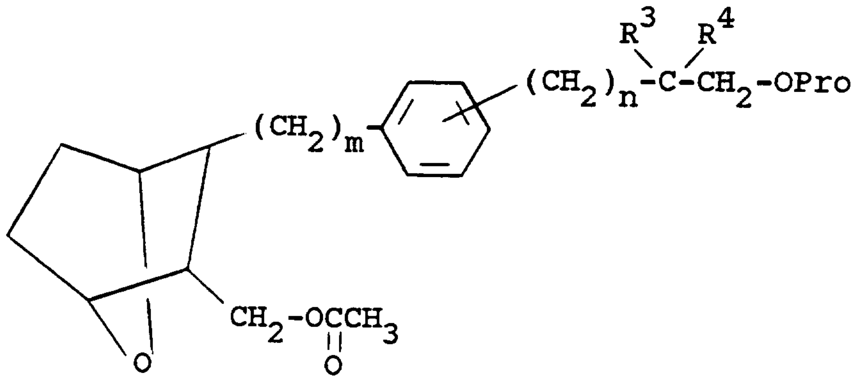

The protected alcohol IIIA is then subjected to a Jones oxidation wherein a solution of protected alcohol IIIA in acetone cooled to from about -l0 to about 25 ° C is treated with Jones reagent (that is, $CrO_3$ dissolved in sulfuric acid in the presence of water, prepared as described in Fieser & Fieser, "Reagents for Organic Synthesis," Vol. I, p. l42 (1967)) to form crude acid which is deacetylated and esterified by treatment with acidic alcohol such as methanolic HCl, to form the alcohol ester IV

IV

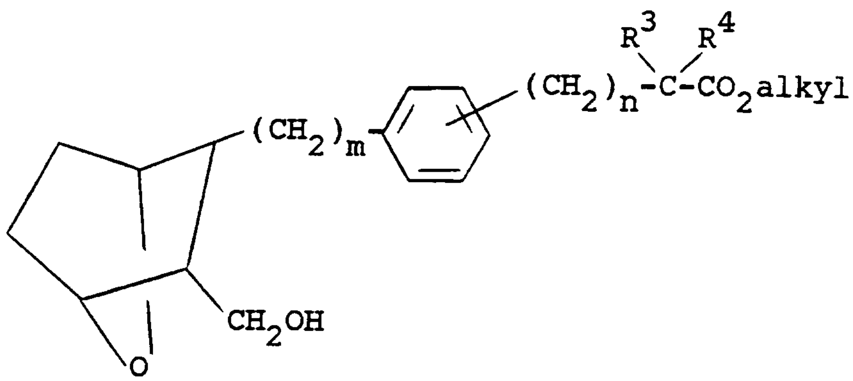

Next, the alcohol ester IV is subjected to a Jones oxidation to form the acid V

V

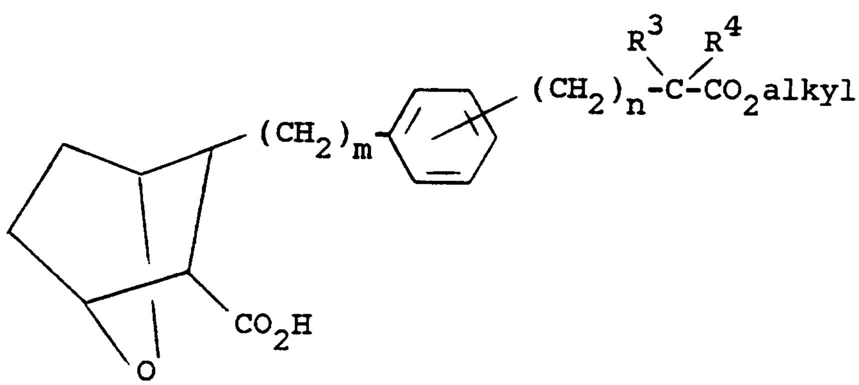

Acid V, in an inert organic solvent, such as tetrahydrofuran or dimethylformamide, is then made to undergo a carbodiimide coupling reaction with amine hydrochloride D

D 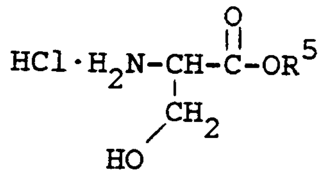

where $R^5$ is benzyl, in the presence of dicyclohexylcarbodiimide (DCC) or l-(3-dimethylaminopropyl)-3-ethylcarbodiimide hydrochloride (WSC) and 1-hydroxybenzotriazole, and triethylamine, under an inert atmosphere such as argon employing a molar ratio of D:V of within the range of from about 1.2:1 to about 1:1, to form amide VI

VI

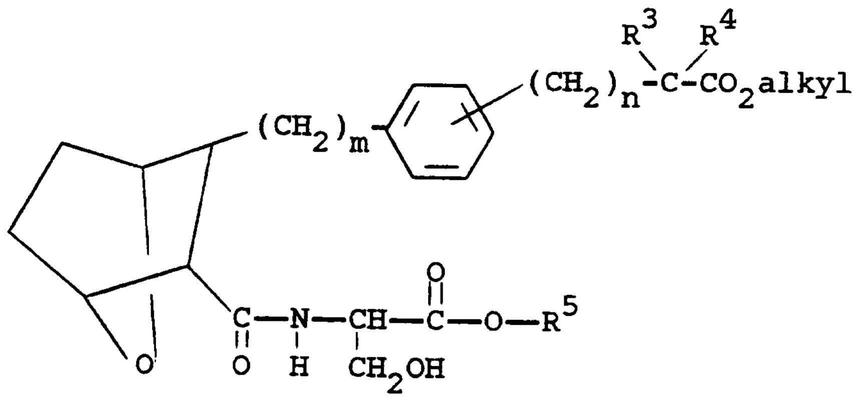

Amide VI is then subjected to cyclodehydration wherein a solution of VI in an inert organic solvent such as tetrahydrofuran, acetonitrile or chloroform, under an inert atmosphere such as argon, is treated with triphenylphosphine (employing a molar ratio of VI:triphenylphosphine of from about 0.5:l to about l:l) and carbon tetrachloride in the presence of an amine base such as triethylamine or diisopropylethylamine, to form oxazoline VII

VII

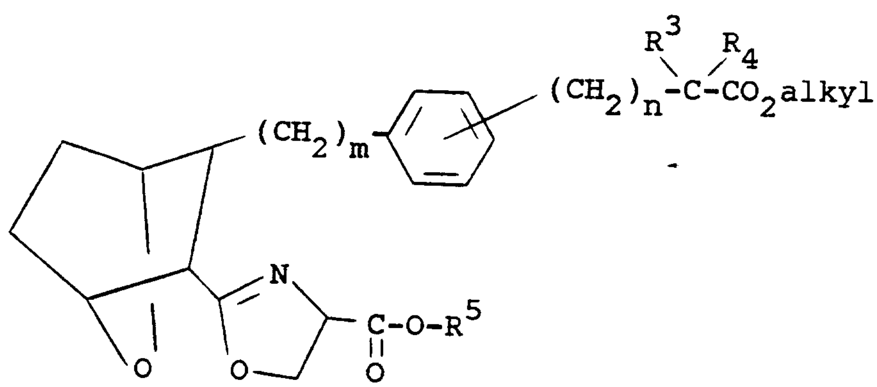

Oxazoline VII is oxidized by treatment with cupric bromide and l,8-diazabicyclo[5.4.0]undec-7-ene (DBU) to form the oxazole VIII

VIII

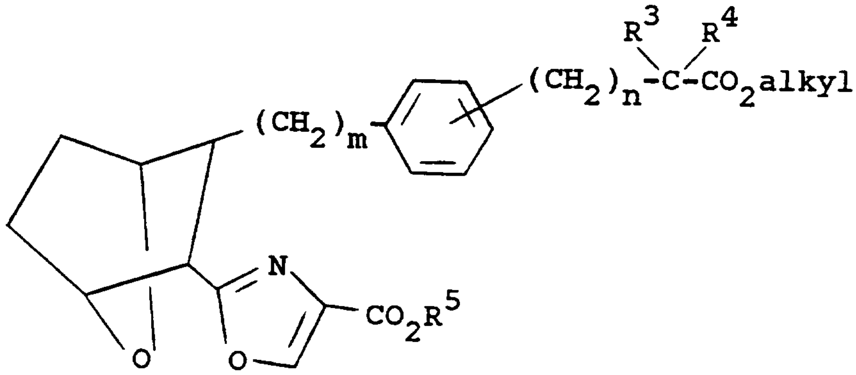

The cupric bromide oxidation is carried out at a temperature of within the range of from about 20 °C to about 50 °C, employing a molar ratio of cupric bromide to VII of within the range of from about 2:l to about 6:l and a molar ratio of cupric bromide to DBU of within the range of from about l:l to about l:3 in an inert organic solvent such as ethyl acetate, methylene chloride or preferably ethylacetate/chloroform (l:l, v/v).

Oxazole VIII is then deprotected to remove $R^5$, for example, by treatment with palladium hydroxide on charcoal and hydrogen in the presence of an inert organic solvent such as ethyl acetate, to form the corresponding acid IX

IX

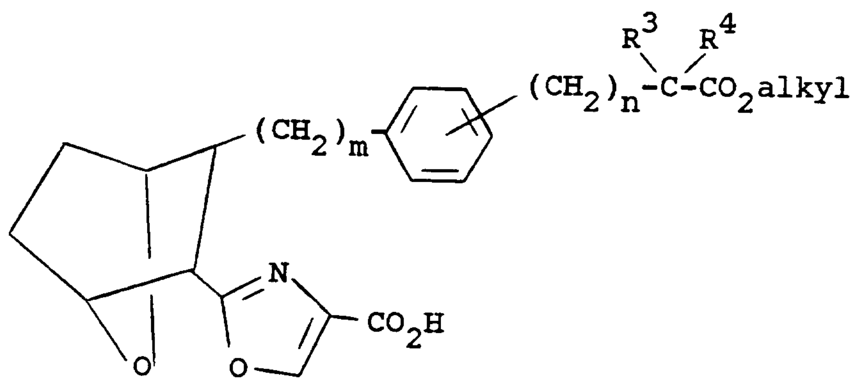

Acid IX is converted to the corresponding acid chloride by treating IX with oxalyl chloride optionally in the presence of catalytic amounts of dimethylformamide, and a solvent such as benzene, toluene or methylene chloride. The so-formed acid chloride is dissolved in an inert organic solvent such as methylene chloride or toluene cooled to a temperature within the range of from about -l0 °C to about +l0 °C, and amine base such as triethylamine or pyridine and amine E, or a salt thereof, are added

E

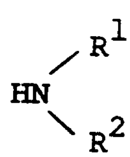

employing a molar ratio of E:IX of within the range of from about 1.1:l to about 1.5:l, to form the oxazole IE

IE

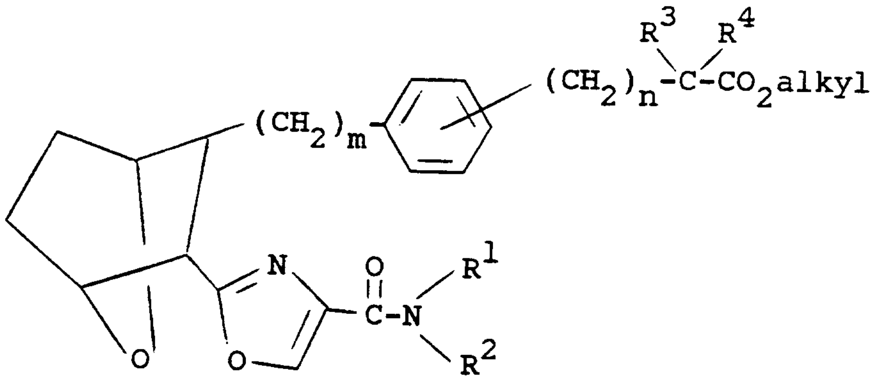

Oxazole X is hydrolyzed to the corresponding acid XI by treating X with a base such as lithium hydroxide, sodium hydroxide or potassium hydroxide to form the corresponding alkali metal salt, followed by neutralization with an acid such as dilute hydrochloric acid or oxalic acid to form acid IF.

IF

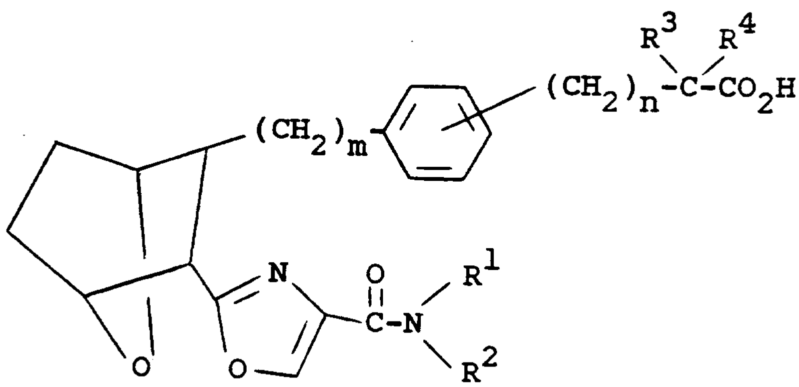

In an alternate preferred procedure for the preparation of IF, acid V is made to undergo a carbodiimide coupling reaction with amine Da

Da

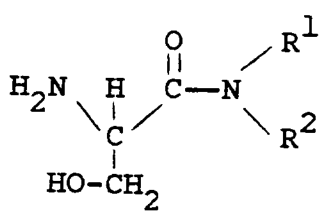

in the presence of dicyclohexylcarbodiimide or l-(3-dimethylaminopropyl)-3-ethylcarbodiimide hydrochloride and l-hydroxybenzotriazole and triethylamine as described hereinbefore to form hydroxy amide VIA

VIA

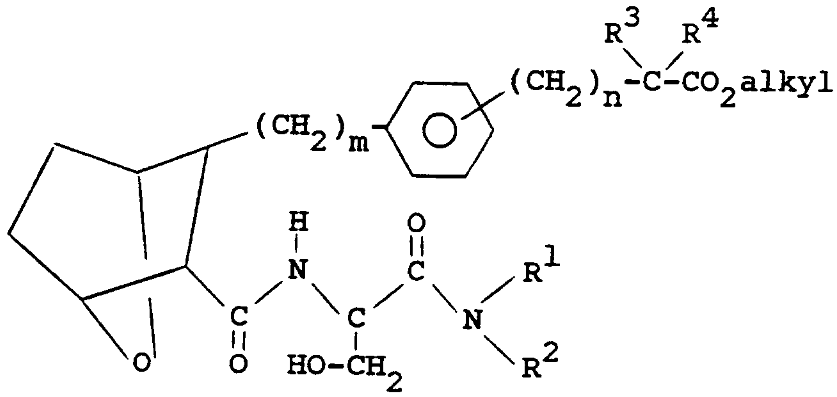

Hydroxy amide VIA is then subjected to cyclodehydration as described hereinbefore (with respect to the preparation of VII). A preferred method for this conversion involves treatment of VIA with an alkylsulfonyl chloride, such as methanesulfonyl chloride in the presence of an amine such as triethylamine or pyridine followed by treatment of the resulting alkylsulfonate intermediate with triethylamine in methylene chloride to form oxazoline VIIA

VIIA

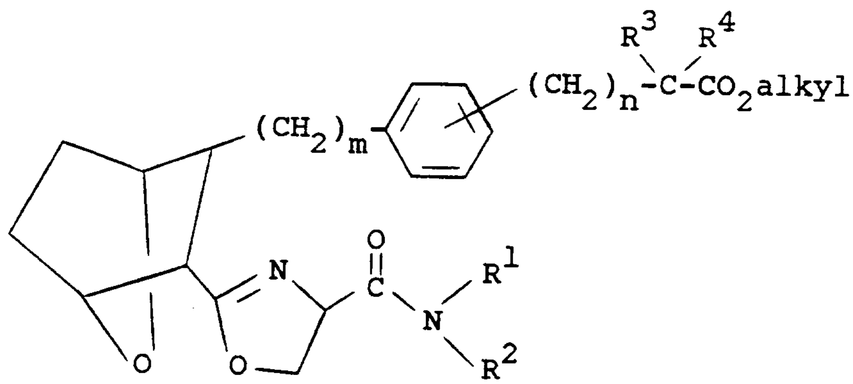

which is made to undergo oxidation as described hereinbefore (with respect to the preparation of VIII) to form oxazole IE.

Ester IF may then be hydrolyzed by treatment with an aqueous solution of alkali metal base and then aqueous acid to form the corresponding acid IF.

Compounds of the invention where X is NH are prepared starting with acid V which is made to undergo a coupling reaction with amine G

G

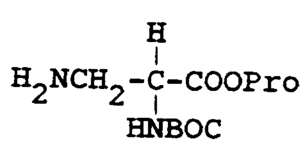

where BOC is t-butyloxycarbonyl and Pro is a protecting group such as benzyl, in the presence of a coupling agent such as l-(3-dimethylaminopropyl)-3-ethylcarbodiimide hydrochloride (WSC) and l-hydroxybenzotriazole (HOBT) and methylene chloride employing a molar ratio of V:G of within the range of from about l.2:l to about l:l, for a period of from about l2 to about 90 hours. The resulting amide is made to undergo a thionation reaction by treating the amide with Lawesson's reagent in the presence of benzene at a temperature of from about 50 to about 75°C for a period of from about l to about 4 hours, to form the ester XXI

XXI

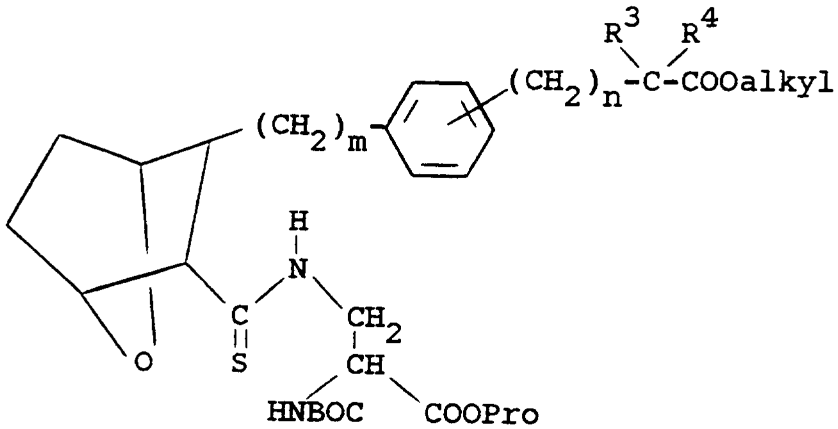

The ester XXI is cyclized by treating a solution of XXI in an inert organic solvent such as acetonitrile, chloroform or tetrahydrofuran with triphenylphosphine (employing a molar ratio of XXI:triphenylphosphine of from about 0.5:l to about l:l) and carbon tetrachloride in the presence of an amine base such as triethylamine or diisopropylethylamine, to form imidazoline XXII

XXII

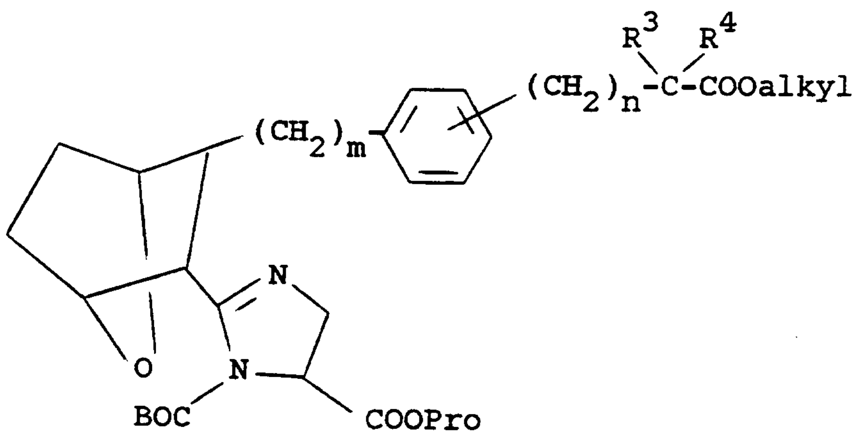

Imidazoline XXII is then deprotected to remove the Pro protecting group, using conventional procedures for example, by hydrogenation when Pro is benzyl, to form the acid XXIII

XXIII

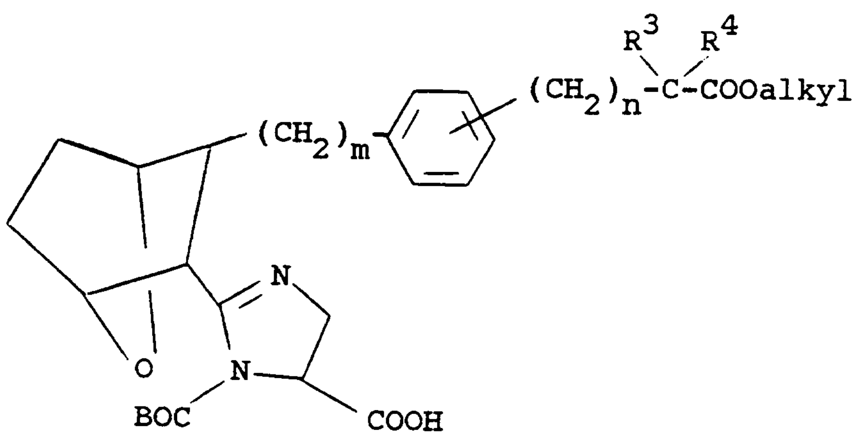

Next, the acid XXIII is made to undergo a coupling reaction with amine E in the presence of an amine base such as pyridine or triethylamine under an inert atmosphere such as argon in the presence of a coupling agent such as WSC and HOBT and chloroform, employing a molar ratio of E:XXIII of within the range of

from about 0.8:l to about l.2:l to form amide XXIV

XXIV

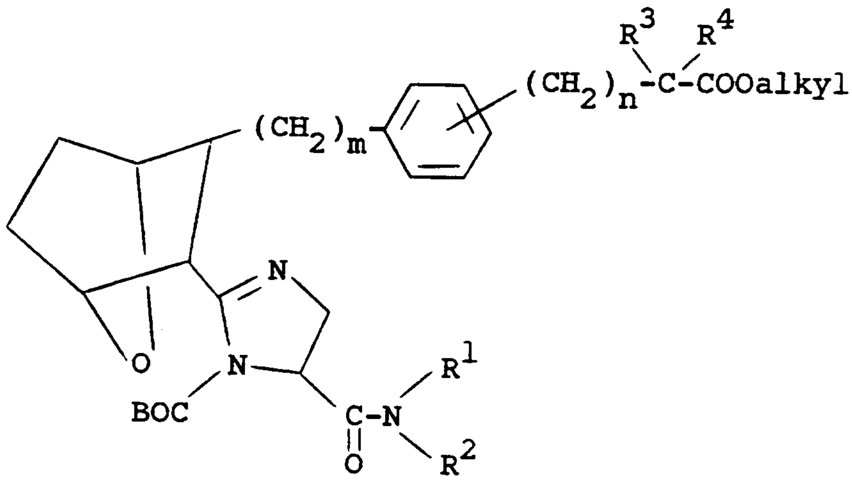

The amine XXIV in solution in methylene chloride is then treated with trifluoroacetic acid to remove the BOC group and form amide XXV

XXV

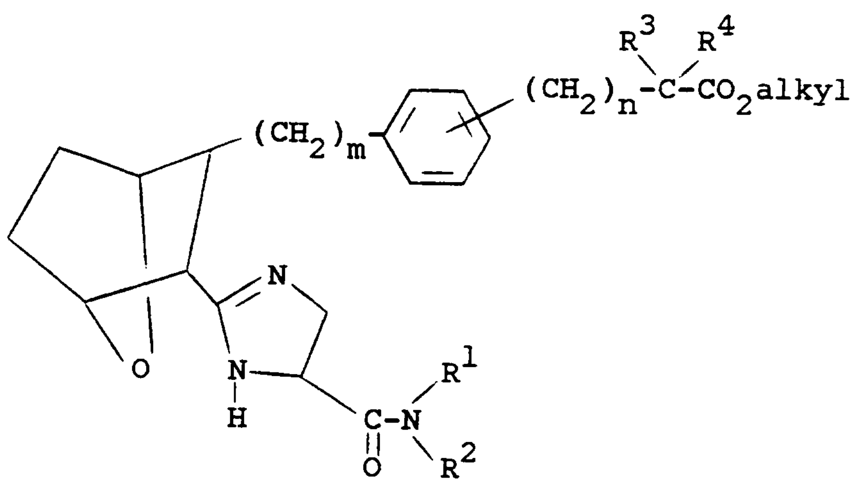

Amide XXV is oxidized by treatment with an oxidizing agent such as manganese dioxide in the presence of an inert organic solvent such as chloroform to form ester IG

IG

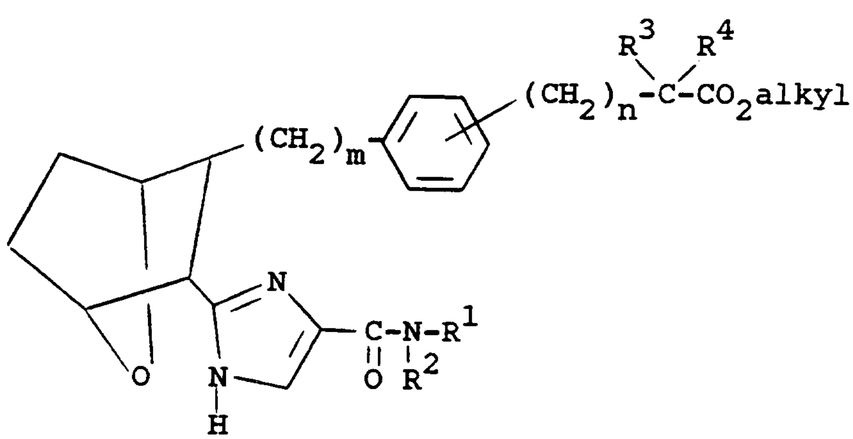

The starting bromophenylalkyl alcohol A where n is l, 2, 3 or 4 may be prepared by alkylating bromide K

K

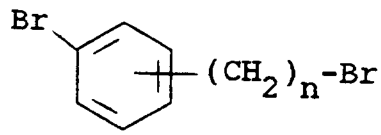

with an ester of the structure J

J

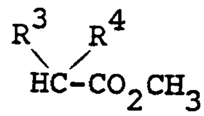

in the presence of lithium diisopropylamine and hexamethylphosphoric amide and an inert organic solvent such as tetrahydrofuran (THF) at reduced temperatures ranging from about -80 °C up to about 0 °C, employing a molar ratio of J:K of within the range of from about l.2:l to about l:l, to form ester L

L

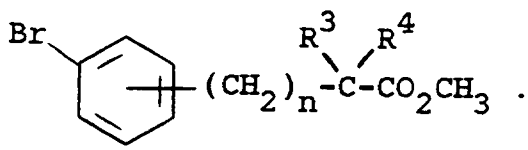

Ester L where n = 0 is prepared via alkylation of aryl ester M using standard methodology familiar

L

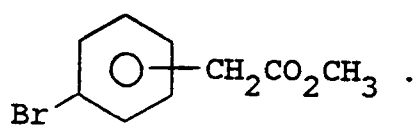

M

to those skilled in the art. Ester L is then hydrolyzed, for example, by treatment with aqueous alkali metal hydroxide and then reduced, for example, by treatment with borane-dimethylsulfide, to form the bromophenylalkyl alcohol A.

The compounds of formula I of the invention wherein Z is -CH = CH or -$(CH_2)_2$- may be prepared as follows.

Compounds of the invention where Z is -CH = CH- and preferably in the cis form, and X is O are prepared starting with the hydroxymethyl compound AA

AA

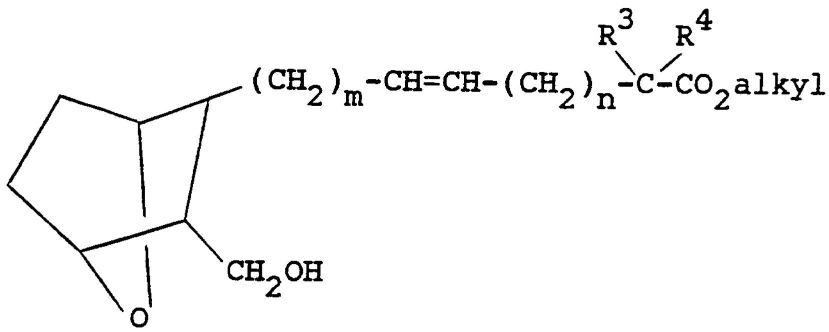

(which is prepared as described in U.S. Patent No. 4,143,054) which is subjected to a Jones oxidation wherein AA is reacted with Jones' Reagent ($CrO_3$ dissolved or suspended in aqueous sulfuric acid), in the presence of acetone, under an inert atmosphere such as argon at a temperature within the range of from about -10 to about 20 °C, to form the corresponding carboxylic acid BB

BB

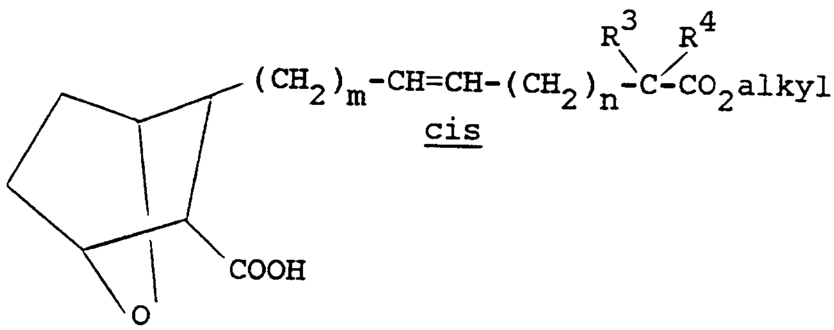

Acid BB, in an inert organic solvent, such as tetrahydrofuran, is then made to undergo a carbodiimide coupling reaction with amide Da

Da

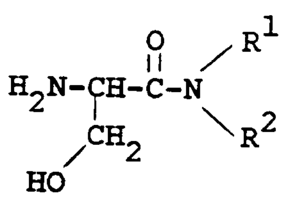

in the presence of dicyclohexylcarbodiimide (DCC) or l-(3-dimethylaminopropyl)-3-ethylcarbodiimide hydrochloride (WSC) and 1-hydroxybenzotriazole under an inert atmosphere such as argon employing a molar ratio of Da:BB of within the range of from about 1.2:1 to about 1:1, to form hydroxybisamide XXX

XXX

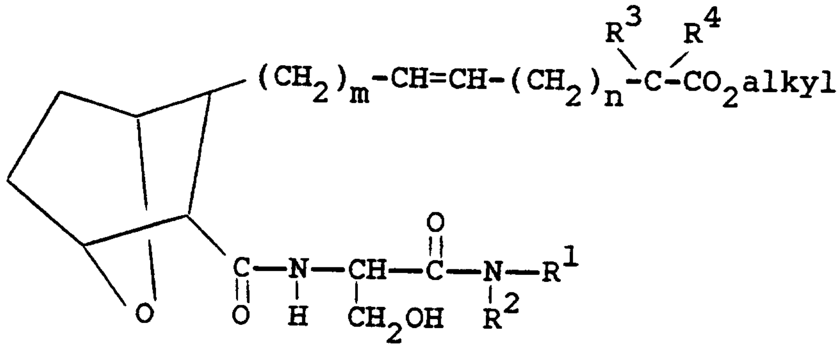

Hydroxybisamide XXX is then subjected to cyclodehydration wherein a solution of XXX in an inert organic solvent such as tetrahydrofuran, acetonitrile or chloroform, under an inert atmosphere such as argon, is treated with triphenylphosphine and carbon tetrachloride in the presence of an amine base such as triethylamine or diisopropylethylamine, to form oxazoline XXXI.

XXXI

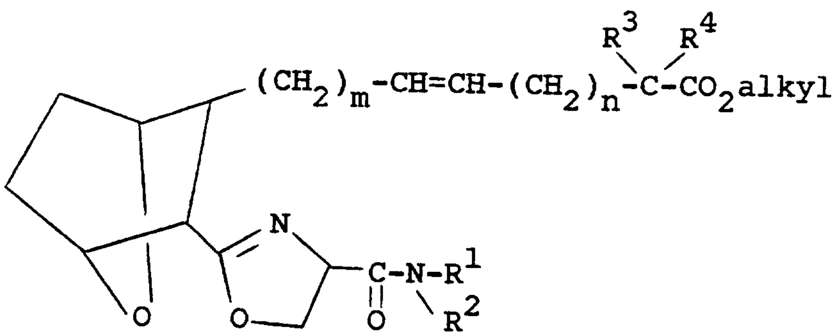

Alternatively, hydroxybisamide XXX is treated with a sulfonyl chloride, such as methane sulfonyl chloride, and an amine base such as triethylamine followed by treatment with potassium carbonate in acetone to form oxazoline XXXI.

Oxazoline XXXI is oxidized by treatment with manganese dioxide or nickel peroxide, preferably nickel peroxide, to form the oxazole IL

IL

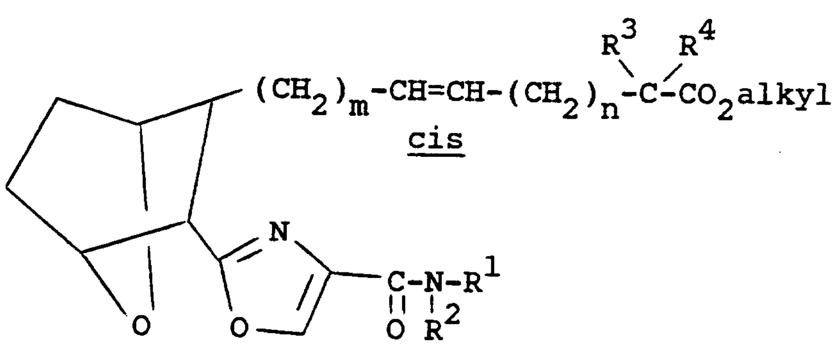

Alternatively, oxazole IDa can be prepared from acid BB

BB

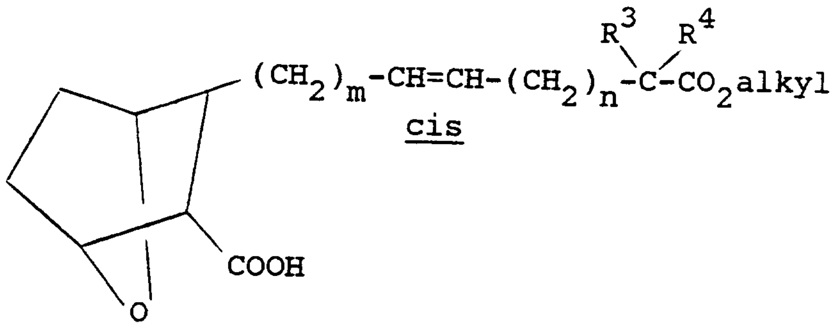

by a carbodiimide coupling as described previously except substituting CC for Da to obtain XXXII.

CC

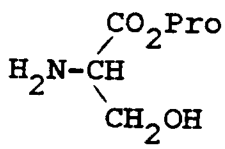

XXXII

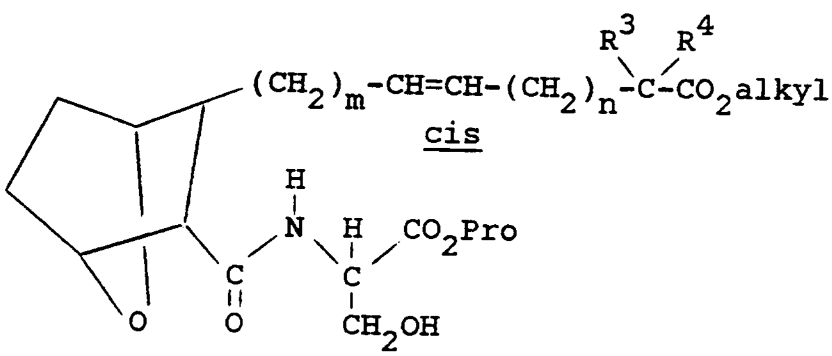

where Pro is a conventional protecting group. Hydroxyamide XXXII is then subjected to a cyclodehydration and oxidation, as described for XXX and XXXI, to form XXXIII

XXXIII

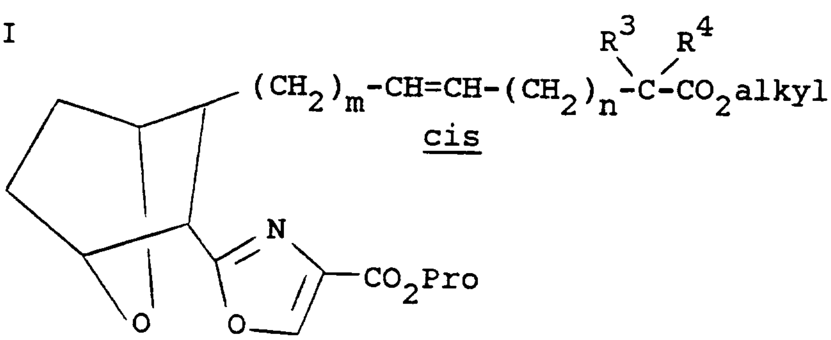

The protecting group of XXXIII can be removed to form the corresponding acid XXXIV

XXXIV

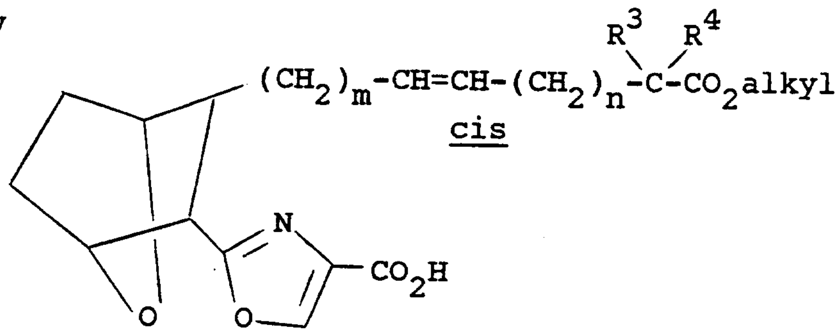

which is treated with excess oxalyl chloride in the presence of an inert organic solvent such as toluene, methylene chloride, or chloroform, and optionally a catalytic amount of dimethylformamide, while stirring under an inert atmosphere such as argon, to form the crude acid chloride XXXV

XXXV

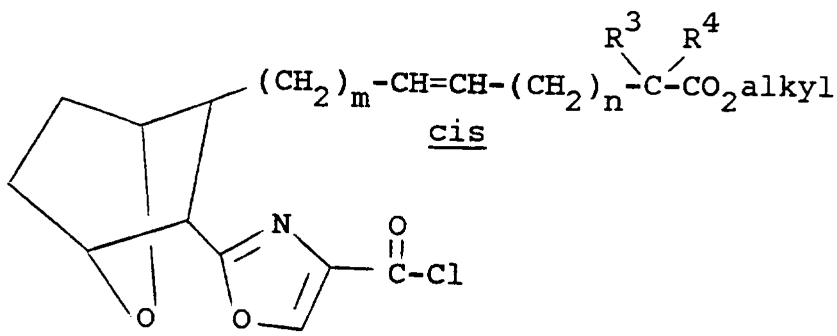

which is treated with amine hydrochloride E'

E'

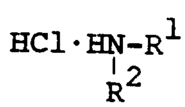

in the presence of an organic base such as triethylamine under an inert atmosphere such as argon, employing a molar ratio of XXXV:E' of within the range of from about 0.5:l to about l:l and preferably from about 0.8:l to about l:l, to form IM.

IM

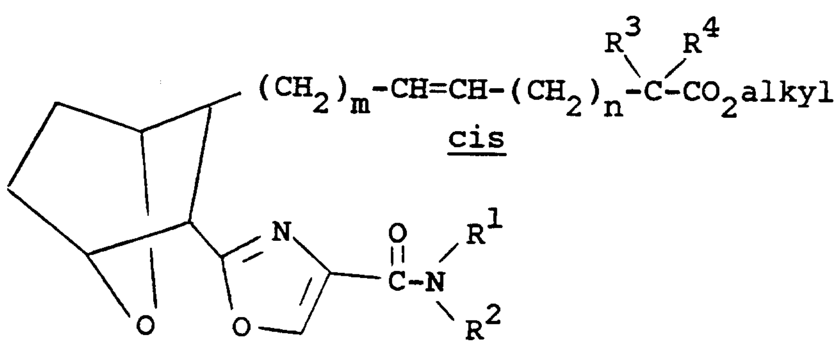

Compounds of the invention IF where X is NH and $Z^1$ is -CH=CH- are prepared starting with acid BB which is made to undergo a coupling reaction with amine G

G

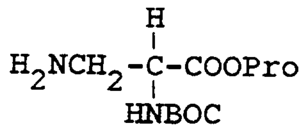

where BOC is t-butyloxycarbonyl and Pro is a protecting group, preferably benzyl, in the presence of a coupling agent such as l-(3-dimethylaminopropyl)-3-ethylcarbodiimide hydrochloride (WSC) and l-hydroxybenzotriazole (HOBT) and methylene chloride employing a molar ratio of BB:Q of within the range of from about l.2:l to about l:l, for a period of from about l2 to about 90 hours. The resulting amide is made to undergo a thionation reaction by treating the amide with Lawesson's reagent in the presence of benzene at a temperature of from about 50 to about 75°C for a period of from about l to about 4 hours, to form the ester XXXVI

XXXVI

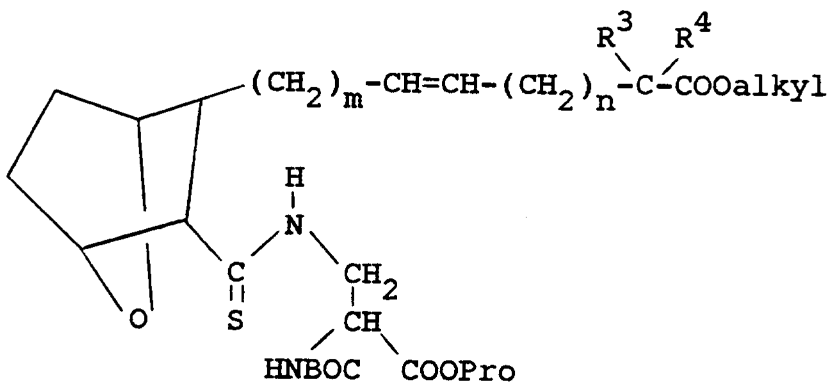

The ester XXXVI is cyclized by treating a solution of XXXVI in an inert solvent such as acetonitrile, chloroform or tetrahydrofuran, with triphenylphosphine (employing a molar ratio of XXXVI:triphenylphosphine of from about 0.8:l to about 0.5:l) and carbon tetrachloride in the presence of an amine base such as triethylamine or diisopropylethylamine, to form imidazoline XXXVII

XXXVII

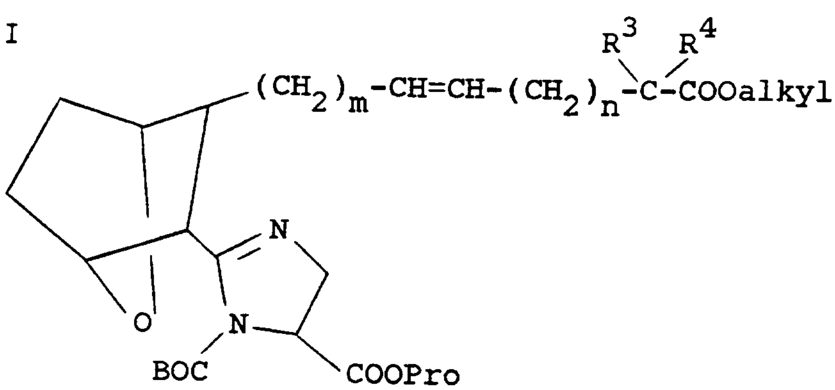

Imidazoline XXXVII is then deprotected to remove the Pro protecting group, using conventional procedures, to form the acid XXXVIII

XXXVIII

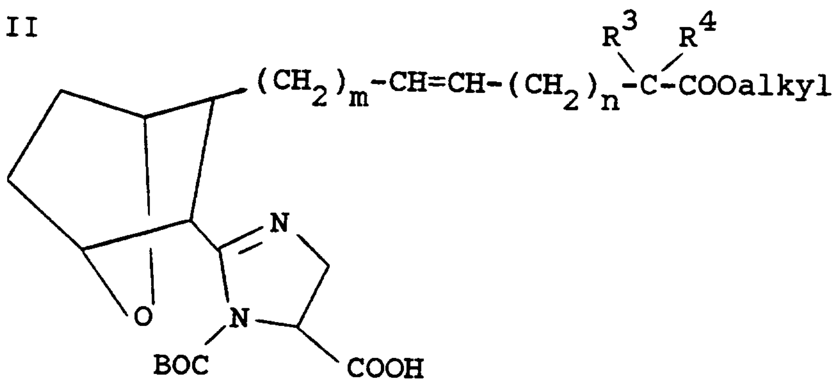

Next, the acid XXXVIII is made to undergo a coupling reaction with amine E in the presence of an amine base such as pyridine or triethylamine under an inert atmosphere such as argon in the presence of a coupling agent such as WSC and HOBT and chloroform, employing a molar ratio of E:XXXVIII of within the range of from about 0.8:l to about l.2:l to form amide XXXIX after removal of the BOC protecting group with trifluoroacetic acid

XXXIX

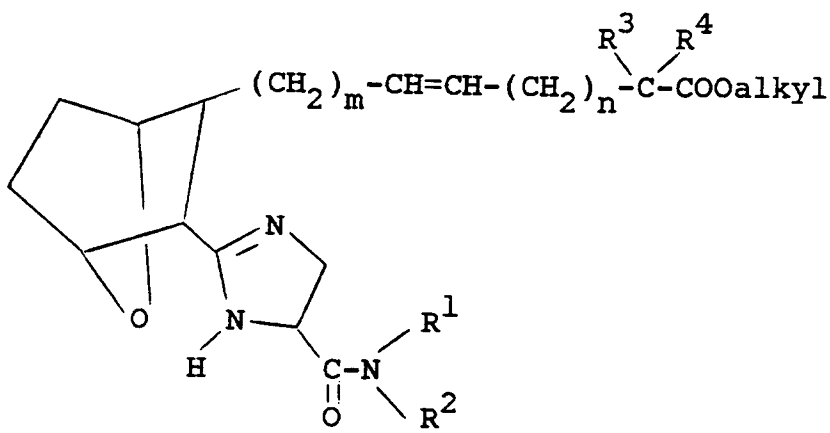

Amide XXXIX is oxidized by treatment with an oxidizing agent such as manganese dioxide in the presence of an inert solvent such as chloroform to form ester IN

IN

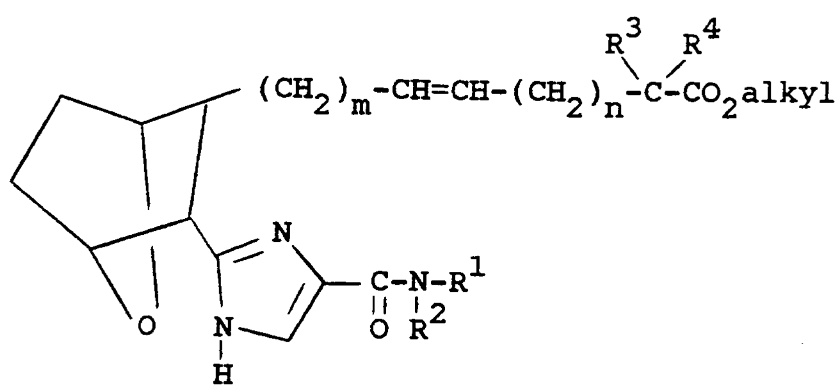

The aforementioned esters of the invention may be converted to the corresponding acids, that is IO

IO

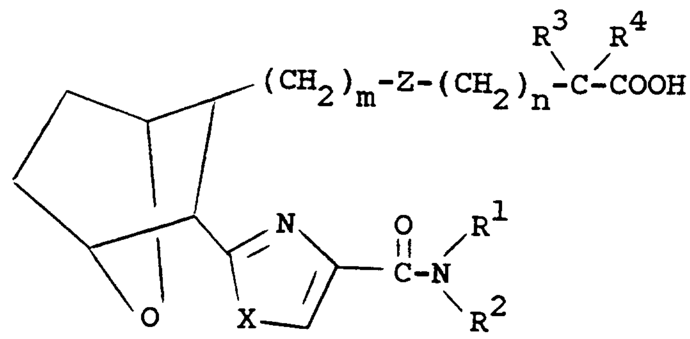

or

IP

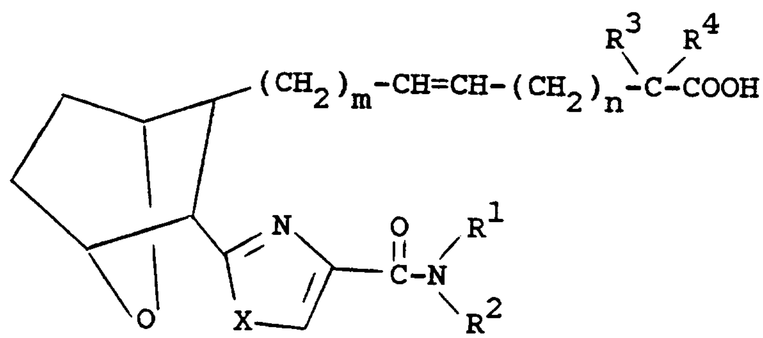

by treating the esters with a base, such as lithium hydroxide, sodium hydroxide or potassium hydroxide to form the corresponding alkali metal salt, followed by neutralization with an acid, such as dilute hydrochloric acid or oxalic acid to form the acid compounds of the invention.

Compounds of formula I wherein Z is $-(CH_2)_2-$may be prepared from acid IP by subjecting IP to hydrogenation using, for example, a hydrogenation catalyst, such as palladium on carbon, in an inert organic solvent such as ethyl acetate (EtOAc) or acetic acid (AcOH) to form the acid of the invention IQ

IQ

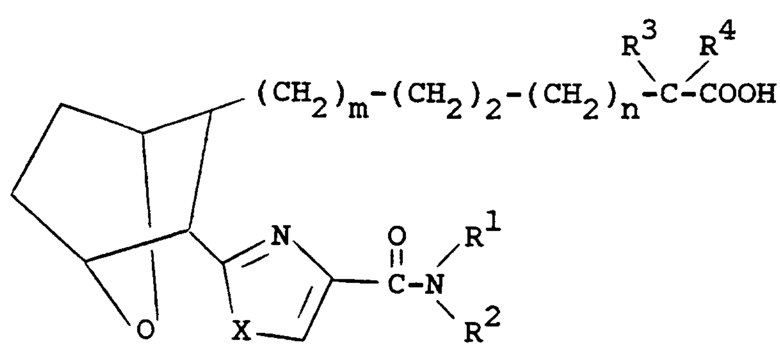

The compounds of this invention have four centers of asymmetry as indicated by the asterisks in formula I. However, it will be apparent that each of the formulae set out above which do not include asterisks still represent all of the possible stereoisomers thereof. All of the various stereoisomeric forms are within the scope of the invention.

The various stereoisomeric forms of the compounds of the invention, namely, cis-exo, cis-endo and all trans forms and stereoisomeric pairs may be prepared by employing starting materials and following the procedures as outlined in U.S. Patent No. 4,143,054. Examples of such stereoisomers are set out below.

Ia

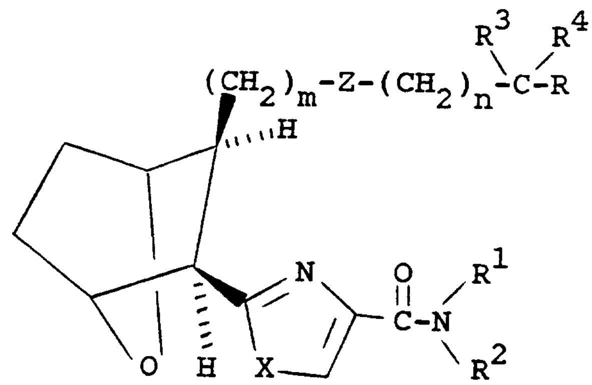

(cis-endo)

Ib

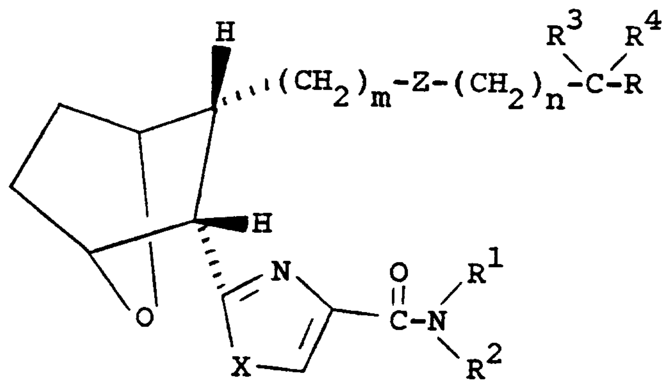

(cis-exo)

Ic

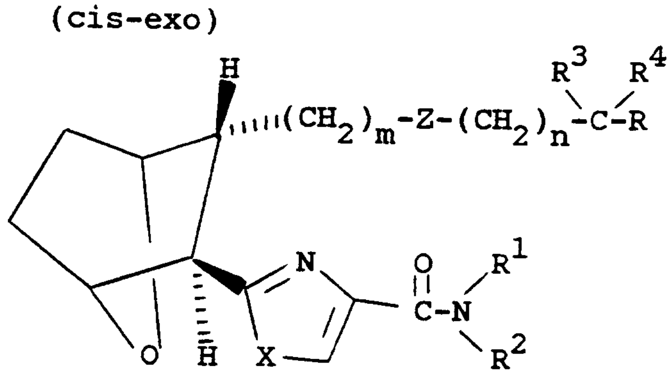

(trans)

Id

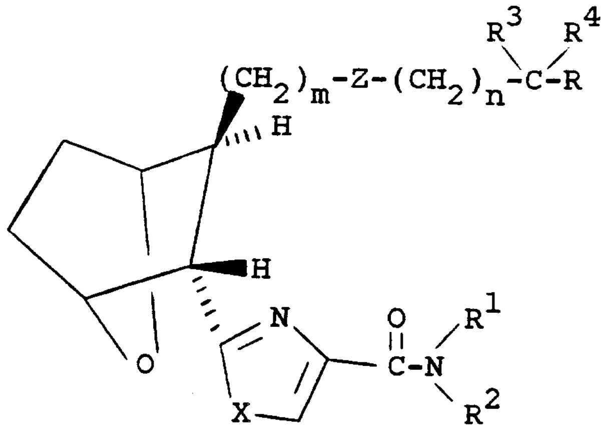

(trans)

The nucleus in each of the compounds of the invention is depicted as

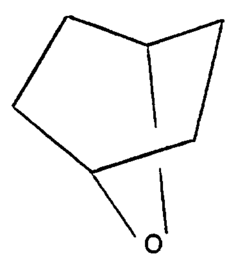

for matter of convenience; it will also be appreciated that the nucleus in the compounds of the invention may be depicted as

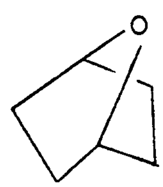

The compounds of this invention are thromboxane receptor antagonists and as such are useful as inhibitors of thromboxane receptor mediated actions. The term "thromboxane receptor antagonist" includes compounds which are so-called thromboxane $A_2$ receptor antagonists, thromboxane $A_2$ antagonists, thromboxane $A_2$/prostaglandin endoperoxide antagonists, TP-receptor antagonists, or thromboxane antagonists.

The compounds of the invention may also be thromboxane synthetase inhibitors and thus may be useful as inhibitors of thromboxane production.

The compounds of this invention are useful as inhibitors of platelet function, i.e., pharmaceutical compositions containing them may be used for the prevention and treatment of thrombotic vascular occlusive disorders, whether complete or partial, for example, arterial thrombosis, including that of the coronary, cerebral, ophthalmic, hepatic, mesenteric, renal, peripheral arteries or vascular or organ grafts, unstable angina, transient ischemic attacks, or intermittent claudication. They may be useful to prevent thrombosis following vascular injury produced in the course of diagnostic or therapeutic procedures such as endarterectomy or angiography. The pharmaceutical compositions may be useful in the treatment or prevention of disorders characterized by platelet consumption and/or activation, including, platelet activation, dysfunction, and/or loss during extracorporeal circulation, the use of radiographic contrast agents, thrombotic thrombocytopenia purpura, disseminated intravascular coagulation, purpura fulminans, hemolytic transfusion reaction, or hemolytic uremic syndrome, systemic lupus, cyclosporine-induced renal toxicity, pulmonary hypertension, side effects from dialysis, or abdominal aortic aneurism repair. The pharmaceutical compositions may be used in the treatment of venous thrombosis or embolism, including pulmonary embolism, deep venous thrombosis, hepatic vein thrombosis, and renal vein thrombosis.

The compounds of this invention are useful as inhibitors of arterial or venous vasoconstriction. Accordingly, pharmaceutical compositions containing them may be useful to prevent vasoconstriction associated with unstable angina, chronic stable angina, and variant, or Prinzmetal's angina, Raynaud's syndrome, migraine headache, vasospasm of the coronary, cerebral, ophthalmic, hepatic, mesenteric, renal, peripheral arteries or vascular grafts, vascular injury such as that associated with surgery or trauma. Hypertension of pregnancy, the hepato-renal syndrome, and pulmonary hypertension are additional examples of vasoconstrictive disorders treatable by the compounds of this invention.

The compounds of this invention are useful as inhibitors of bronchoconstriction, i.e., pharmaceutical compositions containing them may be used in treating airway hyperresponsiveness, allergic bronchospasm, asthma, and bronchoconstrictive responses to environmental, infectious, noxious or mechanical stimuli.

The pharmaceutical compositions containing the compounds of this invention are useful as inhibitors of ischemic and reperfusion injury to various tissues, including myocardium, skin, brain, bowel, or kidney, alone or in combination with other agents intended to restore blood flow. For example, they may be useful for improving postischemic myocardial function and decreasing myocardial infarct size. Ischemia caused by reduced blood flow during diagnostic or therapeutic procedures may benefit by treatment with the pharmaceutical compositions containing the compounds, for example, they reduce the myocardial stunning observed after bypass surgery. In addition, they may be useful for reducing the tissue injury caused by a stroke.

The pharmaceutical compositions containing the compounds of this invention may be useful in the prevention or treatment of other conditions including burns, diabetic retinopathy, tumor metastases and tardive dyskinesia. The compounds may be useful in potentiating diuretic-induced diuresis.

In addition, the thromboxane receptor antagonists of the invention may be used with a thrombolytic agent such as t-PA, streptokinase, urokinase, prourokinase or anisoylated plasminogen-streptokinase activator complex (APSAC) within 6 hours of a myocardial infarction. In such case, the thrombolytic agent may be used in amounts conventionally employed, for example, as disclosed in the Physicians' Desk Reference for reducing post-ischemic myocardial injury.

The pharmaceutical compositions containing the compounds of the invention can be administered orally or parenterally to various mammalian species known to be subject to such maladies, e.g., humans, cats, dogs and the like in an effective amount within the dosage range of the compound of about 0.1 to about 100 mg/kg, preferably about 0.2 to about 50 mg/kg and more preferably about 0.5 to about 25 mg/kg (or from about 1 to about 2500 mg, preferably from about 5 to about 2000 mg) on a regimen in single or 2 to 4 divided daily doses.

The active substance can be utilized in a composition such as tablet, capsule, solution or suspension containing about 5 to about 500 mg per unit of dosage of a compound or mixture of compounds of formula I or in topical form for wound healing (0.01 to 5% by weight compound of formula I, 1 to 5 treatments per day). They may be compounded in conventional matter with a physiologically acceptable vehicle or carrier, excipient, binder, preservative, stabilizer, flavor, etc., or with a topical carrier such as Plastibase (mineral oil gelled with polyethylene) as called for by accepted pharmaceutical practice. Also as indicated in the discussion above, certain members additionally serve as intermediates for other members of the group.

It will be appreciated that the acid or ester compounds of the invention may be converted to any pharmaceutically acceptable salt, employing conventional procedures, to facilitate preparation of pharmaceutical compositions containing such compounds.

The compounds of the invention may be in form of pharmaceutical composition which may also be administered topically to treat peripheral vascular diseases and as such may be formulated as a cream or ointment.

The following Examples represent preferred embodiments of the present invention. Unless otherwise indicated, all temperatures are expressed in degrees Centigrade.

Example I

[IS-(Iα,2α,3α,4α)]-2-[[3-[4-[(Pentylamino)carbonyl]-2-oxazolyl]-7-oxabicyclo[2.2.I]hept-2-yl]methyl]-α,α-dimethylbenzenepropanoic acid, methyl ester

A. 2-Bromo-α,α-dimethylbenzenepropanoic acid, methyl ester

To a solution of I7 mL (I20 mmol, distilled from calcium hydride) of diisopropylamine in I00 mL of dry THF (distilled from sodium/benzophenone) cooled to -78° was added dropwise 44 mL (2.5M in hexanes, II0 mmol, Aldrich) of n-butyllithium solution over ~I5 minutes. The reaction mixture was stirred for an additional 30 minutes then a solution of I0.2 g (I00 mmol, Aldrich) of methyl isobutyrate in I0 mL of THF was added dropwise over I5 minutes. After 30 minutes I7 mL (98 mmol, distilled from calcium hydride) of hexamethyl phosphoric triamide (HMPA) was added followed by a solution of 25.0 g (I00 mmol, Aldrich) of 2-bromobenzyl bromide in I0 mL of THF over ~5 minutes. The reaction mixture was stirred at -78° for I hour (h) then stored at 0° for I8 h. The resulting solution was quenched by addition of 5 mL of water then concentrated in vacuo. The residue was partitioned between I50 mL of IM aqueous HCl solution and I50 mL of diethyl ether(ether). The organic layer was separated, washed with two-I50 mL portions of water, 50 mL of brine, dried (magnesium sulfate) and concentrated in vacuo to give a yellow oil. The crude material was purified by flash chromatography (Merck silica, I20xI0 cm, I:I9 ether/hexane) to afford 22.2 g (8I.9 mmol, 82%) of title ester as a pale yellow liquid.

B. 2-Bromo-α,α-dimethylbenzenepropanol

A solution of 2I.3 g (78.6 mmol) of Part A ester in 50 mL of THF (distilled from sodium/benzophenone) and 50 mL of 3M aqueous NaOH solution was stirred at 65° for I8 h then cooled and concentrated in vacuo. The residue was cooled in an ice-bath and acidified by slow addition of I5 mL of conc. HCl. The resulting slurry was partitioned between I00 mL of water and 50 mL of ether. The aqueous layer was separated and extracted with an additional 50 mL of ether. The ether extracts were combined, washed with 50 mL of brine, dried (magnesium sulfate) and concentrated in vacuo to give I9.8 g of the crude acid as a white solid, mp 84-86°.

To a solution of the crude acid in I00 mL of dry THF (distilled from sodium/benzophenone) cooled in an ice-bath was added dropwise 44 mL (2.0M in THF, 88 mmol, Aldrich) of borane-dimethyl-sulfide solution over 20 minutes. The reaction mixture was stirred at 0° for 2 h then at room temperature for 20 h. The resulting solution was quenched by addition of 5 mL of water, stirred for 30 minutes, then concentrated in vacuo. The residue was partitioned between I00 mL of IM aqueous HCl solution and I00 mL of ether. The organic layer was separated, washed with two-I00 mL portions of IM aqueous NaOH solution, 50 mL of brine, dried (magnesium sulfate) and concentrated in vacuo to give an oil. The oil was dissolved in 50 mL of anhydrous methanol and concentrated in vacuo; repeated with an additional 50 mL of methanol to afford I8.6 g (76.5 mmol, 97% from Part A ester) of title alcohol as a colorless oil.

C. Bromo[[dimethyl(I,I,2-trimethylpropyl)silyl]oxy]benzene

To a solution of I8.6 g (76.5 mmol) of Part B alcohol, I5.0 g (84.3 mmol, Aldrich) of dimethylthexylsilyl chloride and I4 mL (I00 mmol, distilled from calcium hydride) of triethylamine in I00 mL of methylene chloride (distilled from phosphorous pentoxide) was added I.84 g (I5.I mmol, Aldrich) of 4-dimethylaminopyridine at room temperature. The reaction mixture was stirred for 20 h then cooled in an ice-bath and diluted with I00 mL of hexane to precipitate triethylamine hydrochloride. After I5 minutes the slurry was filtered. The filtrate was concentrated in vacuo and the residue partitioned between I0 mL of hexane and I00 mL of IM aqueous HCl solution. The organic layer was separated, washed with an additional I00 mL of IM aqueous HCl solution, I00 mL of water, dried (magnesium sulfate) and concentrated in vacuo to give an oil. The crude material was purified by flash chromatography (Merck silica, I2xI0 cm, I:99 ether/hexane) to afford 23.9 g (62.I mmol, 8I%) of title silyl ether as a colorless liquid.

D. [IS-(Iα,2α(R*),3α,4α)]-α-[2-[3-[[Dimethyl(I,I,2-trimethylpropyl)silyl]oxy]-2,2-dimethylpropyl]phenyl]-7-oxabicyclo[2.2.I]heptane-2,3-dimethanol

To I.00 g (4I mmol, Mallinckrodt) of hammer-crushed magnesium turnings covered with I0 mL of dry THF (distilled from sodium/benzophenone) was added a small crystal of iodine, I50 µL of I,2-dibromoethane

and then ~l/2 of l2.0 g (3l.2 mmol) of title aryl bromide was added in one portion. The mixture was warmed until a reaction started then the remainder of the aryl bromide was added dropwise rapidly followed by heating to reflux (l20° oil bath) for 2 h. The resulting Grignard solution was cooled to room temperature then 20 mL of THF was added to solubilize precipitated reagent.

To a solution of 4.06 g (26.0 mmol) of [3aR-(3aα,4β,7β,7αα)]-octahydro-4,7-epoxyisobenzofuran-l-ol in 20 mL of dry THF cooled in an ice-bath was added dropwise l2.5 mL (2.0M in THF, 25 mmol, Aldrich) of ethylmagnesium bromide over l5 minutes. The reaction mixture was stirred for l5 minutes then the aryl Grignard solution (~3l mmol) from above was added via cannula over ~l0 minutes. The resulting solution was warmed to room temperature, stirred for l8 h then cooled to 0° and quenched by careful addition of 5 mL of water followed by slow addition of 200 mL of l0% aqueous ammonium chloride solution. The mixture was extracted with two-l00 mL portions of ethyl acetate. The organic extracts were combined, washed with 50 mL of brine, dried (magnesium sulfate) and concentrated in vacuo to give an oil. The crude material was purified by flash chromatography (Merck silica, 20x5.0 cm, l:4 ethyl acetate/hexane then 4:l ethyl acetate/hexane) to afford 9.60 g (20.8 mmol, 83%) of title diol as a colorless oil.

E. [lS-(lα,2α,3α,4α)]-2-[[2-[3-[[Dimethyl(l,l,2-trimethylpropyl)silyl]oxy]-2,2-dimethylpropyl]phenyl]methyl]-7-oxabicyclo[2.2.l]heptane-3-methanol

A mixture of 9.50 g (20.6 mmol) of Part D diol and 3.2 g of palladium hydroxide on carbon catalyst (<50% water, Aldrich) in 75 mL of glacial acetic acid was stirred under an atmosphere of hydrogen (balloon) for l8 h. The reaction was filtered to remove the catalyst. The filtrate was concentrated by rotoevaporation (room temperature bath/oil pump vacuum) to give an oil. The oil was dissolved in 50 mL of toluene and concentrated in vacuo to remove residual acetic acid; repeated with an additional 50 mL of toluene. The crude material was purified by flash chromatography (Merck silica, 20x5.0 cm, 2:3 ethyl acetate/hexane) to afford 8.23 g (l8.5 mmol, 90%) of title alcohol as a colorless oil.

F. [lS-(lα,2α,3α,4α)]-2-[[2-[3-[[Dimethyl(l,l,2-trimethylpropyl)silyl]oxy]-2,2-dimethylpropyl]phenyl]methyl]-7-oxabicyclo[2.2.l]heptane-3-methanol, acetate

To a solution of 8.20 g (l8.4 mmol) of Part E alcohol in 20 mL of pyridine (Burdick and Jackson) and 20 mL of reagent acetic anhydride (Mallinckrodt) was added at room temperature ll2 mg (0.92 mmol, Aldrich) of 4-dimethylaminopyridine. The reaction mixture was stirred for l h then concentrated in vacuo and the residue partitioned between 50 mL of ethyl acetate and 50 mL of ice-cold lM aqueous HCl solution. The organic layer was separated, washed with an additional 50 mL of lM aqueous HCl solution, 50 mL of water, 25 mL of brine, dried (magnesium sulfate) and concentrated in vacuo to give 8.83 g (l8.l mmol, 98%) of title acetate as a colorless oil.

G. [lS-(lα,2α,3α,4α)]-2-[[3-(Hydroxymethyl)-7-oxabicyclo[2.2.l]hept-2-yl]methyl-(α,α-dimethylbenzenepropanoic acid, methyl ester

To a solution of 8.80 g (l8.0 mmol) of Part F acetate in l00 mL of reagent acetone in an ambient water bath was added rapidly 30 mL (2.6M in $Cr^{+6}$, prepared as described in Fieser and Fieser, "Reagents for Organic Synthesis," Vol.l, p. l42) of Jones reagent. The reaction mixture was stirred for 2 h then quenched by addition of 5 mL of isopropanol followed by stirring for l5 minutes. The resulting slurry was filtered through a pad of Celite to remove precipitated chromium salts. The filtrate was concentrated in vacuo and the residue partitioned between 50 mL of ether and l00 mL of lM aqueous HCl solution. The aqueous layer was separated and extracted with an additional 50 mL of ether. The organic layers were combined, washed with 50 mL of brine, dried (magnesium sulfate) and concentrated in vacuo to give the crude acid-acetate as an oil. To the crude material was added l00 mL of an ice-cold solution of acidic methanol (prepared by addition of 2 mL of acetyl chloride to l00 mL of anhydrous methanol at 0°) then stirred at room temperature for 64 h. The solution was concentrated in vacuo and the resulting oil purified by flash chromatography (Merck silica, 20x5.0 cm, 3:2 ethyl acetate/hexane) to afford 4.3l g (l3.l mmol, 73%) of title alcohol-ester as a colorless oil.

H. [IS-(lα,2α,3α,4α)]-2-[(3-Carboxy-7-oxabicyclo[2.2.l]hept-2-yl)methyl]-α,α-dimethylbenzenepropanoic acid, methyl ester

To a solution of 2.00 g (6.06 mmol) of Part G alcohol-ester in 25 mL of reagent acetone in an ambient water bath was added dropwise 6.0 mL (2.6M in $Cr^{+6}$) of Jones reagent. The reaction mixture was stirred for l.5 h then quenched by addition of 5 mL of isopropanol and stirred for l5 minutes. The resulting slurry was filtered through a pad of Celite to remove precipitated chromium salts. The filtrate was concentrated in vacuo and the residue partitioned between 50 mL of ether and 50 mL of lM aqueous HCl solution. The aqueous layer was separated and extracted with an additional 50 mL of ether. The organic layers were combined, washed with 50 mL of brine, dried (magnesium sulfate) and concentrated in vacuo to give an oil. The crude material was purified by flash chromatography (Merck silica, l5x5.0 cm, l:l9 methanol/methylene chloride) to afford l.92 g (5.58 mmol, 92%) of title acid as a colorless glass.

I. [IS-[lα,2α,3α(R*),4α]]-2-[[3-[[[l-(Hydroxymethyl)-2-oxo-2-(phenylmethoxy)ethyl]amino]carbonyl]-7-oxabicyclo[2.2.l]hept-2-yl]methyl]-α,α-dimethylbenzenepropanoic acid, methyl ester

To a solution of l.78 g (5.l7 mmol) of Part H acid in l5 mL of DMF (Burdick and Jackson) cooled in an ice-bath was added 960 mg (80%, 5.7 mmol, Aldrich) of l-hydroxybenzotriazole hydrate, l.32 g (5.69 mmol, Sigma) of L-serine benzyl ester hydrochloride, 0.80 mL (5.7 mmol, distilled from calcium hydride) of triethylamine then l.09 g (5.69 mmol, JBL) of WSC. The reaction mixture was stirred at 0° for 2 h then at room temperature for l6 h. The resulting slurry was partitioned between 30 mL of ethyl acetate and 60 mL of lM aqueous HCl solution. The aqueous layer was separated and extracted with an additional 30 mL of ethyl acetate. The organic extracts were combined, dried (magnesium sulfate) and concentrated in vacuo to give an oil. The crude material was purified by flash chromatography (Merck silica, 20x5.0 cm, ethyl acetate) to afford 2.50 g (4.80 mmol, 93%) of title amide as a colorless glass.

J. [IS-(lα,2α,3α,4α)]-2-[[3-[4,5-Dihydro-4-[(phenylmethoxy)carbonyl]-2-oxazolyl]-7-oxabicyclo[2.2.l]hept-2-yl]-methyl]-α,α-dimethylbenzenepropanoic acid, methyl ester

A mixture of 2.45 g (4.70 mmol) of Part I amide and l.85 g (7.06 mmol, Aldrich) of triphenylphosphine in 20 mL of acetonitrile (Burdick and Jackson) was stirred at room temperature until homogeneous then l.25 mL (7.2 mmol, Aldrich) of diisopropylethylamine and 0.70 mL (7.2 mmol, Mallinckrodt) of reagent carbon tetrachloride were added. The reaction mixture was stirred for 2.5 h then partitioned between 75 mL of saturated sodium bicarbonate solution and 25 mL of ethyl acetate. The aqueous layer was separated and extracted with an additional 25 mL of ethyl acetate. The organic extracts were combined, dried (sodium sulfate) and concentrated in vacuo to give an oily solid. The crude material was purified by flash chromatography (Merck silica, 20x5.0 cm, 2:l ethyl acetate/hexane) to afford 2.05 g (4.08 mmol, 87%) of title oxazoline as a solid.

K. [IS-(lα,2α,3α,4α)]-α,α-Dimethyl-2-[[3-[4-[(phenylmethoxy)carbonyl]-2-oxazolyl]-7-oxabicyclo[2.2.l]hept-2-yl]methyl]benzenepropanoic acid, methyl ester

To a solution of 2.66 g (l7.5 mmol, Aldrich) of DBU in 20 mL of ethyl acetate (Burdick and Jackson) was added l.95 g (8.74 mmol, Aldrich) of copper(II) bromide. After l0 minutes a solution of 2.00 g (3.98 mmol) of Part J oxazoline in 20 mL of chloroform (Burdick and Jackson) was added over 5 minutes. The addition was mildly exothermic. The reaction mixture was stirred for l6 h then added was l00 mL of l:l saturated aqueous ammonium chloride/concentrated ammonium hydroxide solution and extracted with 50 mL of ether. The aqueous layer was separated and extracted with an additional 25 mL of ether. The combined ether layers were washed with 50 mL of brine, dried (magnesium sulfate) and concentrated in vacuo to give an oil. The crude material was purified by flash chromatography (Merck silica, 20x5.0 cm, l:l ethyl acetate/hexane) to give 645 mg (l.29 mmol, 32%) of title oxazole as a white solid, mp 89-9l°.

L. [IS-(lα,2α,3α,4α)]-2-[[3-(4-Carboxy-2-oxazolyl)-7-oxabicyclo[2.2.l]hept-2-yl]methyl]-α,α-dimethylbenzenepropanoic acid, methyl ester

A mixture of 640 mg (l.28 mmol) of Part K benzyl ester and 64 mg of 20% palladium hydroxide on carbon catalyst (<50% water, Aldrich) in l0 mL of ethyl acetate was stirred rapidly under an atmosphere of hydrogen (balloon) for 2 h. The reaction mixture was passed through a 0.4 µM polycarbonate membrane to

remove the catalyst. The filtrate was concentrated in vacuo to afford 490 mg (l.l9 mmol, 93%) of title oxazole acid as a white solid, mp l29-l3l°.

M. [lS-(lα,2α,3α,4α)]-2-[[3-[4-[(Pentylamino)carbonyl]-2-oxazolyl]-7-oxabicyclo[2.2.l]hept-2-yl]methyl]-α,α-dimethylbenzenepropanoic acid, methyl ester

To a solution of 300 mg (0.73 mmol) of Part L oxazole acid in 3 mL of methylene chloride (distilled from phosphorous pentoxide) was added at room temperature a small drop of DMF then 85 μL (0.97 mmol, Aldrich) of oxalyl chloride (gas evolution). The solution was stirred for 20 minutes then concentrated in vacuo to give the crude acid chloride as a foam.

To a solution of the crude acid chloride (~0.73 mmol) in 2 mL of dry methylene chloride cooled in an ice-bath was added dropwise a solution of 87 mg (l.0 mmol, Aldrich) of n-amylamine and l0l mg (l.0 mmol, distilled from calcium hydride) of triethylamine in 2 mL of methylene chloride. The reaction mixture was stirred for l0 minutes then partitioned between 20 mL of ethyl acetate and 20 mL of lM aqueous HCl solution. The organic layer was separated, washed with 20 mL of brine, dried (magnesium sulfate) and concentrated in vacuo to give a solid. The crude solid was purified by flash chromatography (Merck silica, l5x3.0 cm, 2:l ethyl acetate/hexane) to afford 33l mg (0.69 mmol, 94%) of title compound as a white solid, mp l30-l3l°.

## Example 2

[lS-(lα,2α,3α,4α)]-2-[[3-[4-[(Pentylamino)carbonyl]-2-oxazolyl]-7-oxabicyclo[2.2.l]hept-2-yl]methyl]-α,α-dimethylbenzenepropanoic acid

A mixture of 280 mg (0.58 mmol) of Example l methyl ester and 840 mg (20 mmol, Aldrich) of lithium hydroxide monohydrate in l0 mL of l:l p-dioxane/water was stirred rapidly at 55° for 3 h. The reaction mixture was cooled in an ice-bath and acidified by dropwise addition of 2 mL of concentrated HCl then partitioned between 20 mL of water and 20 mL of ethyl acetate. The aqueous layer was separated and extracted with an additional 20 mL of ethyl acetate. The combined organic layers were dried (magnesium sulfate) and concentrated in vacuo to give a solid. The crude solid was recrystallized (ethylacetate/hexane) to afford 225 mg (0.40 mmol, 83%) of title acid as a white solid, mp l39-l40°.

IR(KBr): 3400 (broad), 2959, 2934, l7l7, l638, l603, l526 $cm^{-l}$.

MS(CI): 469 $(M+H)^+$.

OR: $[\alpha]_D = +33°$ (c=0.25 in chloroform).

TLC: $R_f$ (silica gel, l:9 methanol/methylene chloride)=0.58, ammonium molybdate/ceric sulfate and UV, homogeneous.

| Analysis Calc'd for $C_{27}H_{36}N_2O_5$: | | | |
|---|---|---|---|
| | C, 69.2l; | H, 7.74; | N, 5.98 |
| Found: | C, 69.3l; | H, 7.64; | N, 6.07. |

## Example 3

[lS-(lα,2α,3α,4α)]-2-[[3-[4-[(4-Cyclohexylbutyl)carbonyl]-2-oxazolyl]-7-oxabicyclo[2.2.l]hept-2-yl]methyl]-α,α-dimethylbenzenepropanoic acid, methyl ester

To a solution of 89 mg (0.2l mmol) of Example l, Part L oxazole acid in 2 mL of methylene chloride (distilled from phosphorous pentoxide) at room temperature was added a small drop of DMF then 25 μL (0.28 mmol, Aldrich) of oxalyl chloride. The reaction mixture was stirred until gas evolution ceased (~l5 minutes) then concentrated in vacuo to give the crude acid chloride as a pale yellow oil.

To a solution of the acid chloride (~0.2l mmol) in 2 mL of dry methylene chloride cooled in an ice-bath, was added dropwise a solution of 39 mg (0.25 mmol) of 4-cyclohexylbutylamine and 30 mg (0.30 mmol, distilled from calcium hydride) of triethylamine in l mL of dry methylene chloride. The reaction mixture was stirred for l0 minutes then partitioned between l5 mL of lM aqueous HCl solution and l5 mL of ethyl acetate. The aqueous layer was separated and extracted with an additional l5 mL of ethyl acetate. The organic layers were combined, dried (magnesium sulfate) and concentrated in vacuo to give a solid. The crude

material was purified by flash chromatography (Merck silica, l2xl.5 cm, 2:l ethyl acetate/hexane) to afford ll5 mg (0.2l mmol, l00%) of title ester as a white solid, mp l48-l49°.

Example 4

[lS-(lα,2α,3α,4α)]-2-[[3-[4-[[(4-Cyclohexylbutyl)amino]carbonyl]-2-oxazolyl]-7-oxabicyclo[2.2.l]hept-2-yl]-methyl]-α,α-dimethylbenzenepropanoic acid

A mixture of ll0 mg (0.20 mmol) of Example 3 ester and 630 mg (l5 mmol, Aldrich) of lithium hydroxide in 7.5 mL of 2:l p-dioxane/water was stirred rapidly at 55° for 5 h. The reaction mixture was cooled in an ice-bath, acidified with 20 mL of lM aqueous HCl solution then extracted with two-20 mL portions of ethyl acetate. The organic extracts were combined, dried (magnesium sulfate) and concentrated in vacuo to give a solid. The crude material was recrystallized (hot acetonitrile) to afford 80 mg (0.l5 mmol, 75%) of title acid as a white solid, mp l33-l35°C.

Example 5

[lS-(lα,2α,3α,4α)]-2-[[3-[4-[[(4-Cyclohexylbutyl)amino]carbonyl]-lH-imidazol-2-yl]-7-oxabicyclo[2.2.l]hept-2-yl]-methyl]-α,α-dimethylbenzenepropanoic acid, methyl ester

A. 3-Amino-2-[[(l,l-dimethylethoxy)carbonyl]amino]propanoic acid, benzyl ester

To a stirred mixture of [bis(trifluoroacetoxy)iodosylbenzene (2.00 g, 4.66 mmol) in 24 mL of l:l DMF-water was added N-α-Boc-asparagine benzyl ester (l.00 g, 3.ll mmol, preparation was described by Wang, G. et al, in J. Org. Chem., Vol, 42, p l286-l290, l977). This mixture was stirred in a cold water bath for l5 minutes at which time dry pyridine (0.50 mL, 6.2l mmol) was added. The mixture was stirred at room temperature for 4 hours and concentrated in vacuo. The crude product was partitioned between l0 mL of lN HCl solution and ether (4 X l5 mL). The aqueous layer was neutralized with $NaHCO_3$, saturated with NaCl and extracted with EtOAc (4 X l5 mL). The combined EtOAc extracts were dried ($MgSO_4$), filtered and concentrated in vacuo to give 0.53 g (58%) of title amine.
TLC: silica gel, 6% $CH_3OH/CH_2Cl_2$, $R_f$ 0.44, $Ce(SO_4)_2$.

B. [lS-[lα,2α,3α,4α]]-2-[[3-[[[2-[[(l,l-Dimethylethoxy)carbonyl]amino]-3-oxo-3-(phenylmethoxy)propyl]amino]-oxomethyl]-7-oxabicyclo[2.2.l]hept-2-yl]methyl]-α,α-dimethylbenzenepropanoic acid, methyl ester

To a stirred mixture of Example l, Part H acid (ll.8 mmol), l-hydroxybenzotriazole monohydrate (ll.8 mmol) and Part A amine (ll.8 mmol) in dry DMF under argon at 0°C is added sequentially $(C_2H_5)_3N$(23.6 mmol) and ethyl-3-(3-dimethylamino)propyl carbodiimide hydrochloride salt (ll.8 mmol). The mixture is stirred at room temperature for l2 hours and concentrated in vacuo. The crude product is diluted with EtOAc and washed with 0.lN NaOH solution, lN HCl solution, saturated $NaHCO_3$ solution and brine. The EtOAc layer is dried ($MgSO_4$), filtered and concentrated in vacuo. This is chromatographed on Merck silica gel 60 to give title amide.

C. [lS-[lα,2α,3α,4α]]-2-[[3-[[[2-[[(l,l-Dimethylethoxy)carbonyl]amino]-3-oxo-3-(phenylmethoxy)propyl]amino]-thioxomethyl]-7-oxabicyclo[2.2.l]hept-2-yl]methyl]-α,α-dimethylbenzenepropanoic acid, methyl ester

To a stirred mixture of Part B amide (l.l2 mmol) in l4 mL benzene under argon is added Lawesson's reagent (0.72 mmol). The mixture is heated at 65°C under argon for 2 hours and cooled to room temperature. The mixture is diluted with ether and washed with saturated $NaHCO_3$ solution and brine. The organic layer is dried ($MgSO_4$), filtered and concentrated in vacuo. Purification is effected by flash chromatography on Merck silica gel 60 to give title thioamide.

D. [lS-[lα,2α,3α,4α]]-2-[[3-[l-[(l,l-Dimethylethoxy)carbonyl]-4,5-dihydro-5-[(phenylmethoxy)carbonyl]-lH-imidazol-2-yl]-7-oxabicyclo[2.2.l]hept-2-yl]methyl]-α,α-dimethylbenzenepropanoic acid, methyl ester

To a stirred mixture of Part C thioamide (0.57 mmol), $(C_6H_5)_3P$ (l.7l mmol) and $(C_2H_5)_3N$ (l.7l mmol) in acetonitrile is added $CCl_4$ (6.27 mmol). The mixture is stirred at room temperature for 4 hours and diluted with ether and water. The resulting mixture is saturated with NaCl and extracted with ether. The combined

ether extracts are dried ($MgSO_4$), filtered and concentrated in vacuo. This is chromatographed on Merck silica gel 60 to give title Boc (or BOC)-imidazoline.

E. [lS-[lα,2α,3α,4α]]-2-[[3-[5-[[(4-Cyclohexylbutyl)amino]carbonyl]-[l-[(l,l-dimethylethoxy)carbonyl]-4,5-dihydro-lH-imidazol-2-yl]-7-oxabicyclo[2.2.l]hept-2-yl]methyl]-α,α-dimethylbenzenepropanoic acid, methyl ester

To a stirred mixture of Part D Boc-imidazoline (0.32 mmol) in methanol under argon is added 20% Pd/C (20% based on the weight of Part D compound). The atmosphere is replaced with hydrogen by several vacuum-fill cycles. The mixture is stirred at room temperature for 4.5 hours and the catalyst is filtered off through a 0.4 μm polycarbonate film. The catalyst is rinsed with DMF. The filtrate is concentrated in vacuo to give crude acid. To a stirred mixture of this acid, l-hydroxybenzotriazole monohydrate (0.32 mmol) and 4-cyclohexylbutyl amine hydrochloride salt (0.38 mmol) in DMF under argon at 0°C is added sequentially $(C_2H_5)_3N$ (0.79 mmol) and ethyl-3-(3-dimethylamino)propyl carbodiimide hydrochloride salt (0.32 mmol). The mixture is stirred at room temperature for l8 hours and concentrated in vacuo. The crude product is partitioned between EtOAc and 0.lN NaOH solution, lN HCl solution and saturated $NaHCO_3$ solution. The organic layer is dried ($MgSO_4$), filtered and concentrated in vacuo. This is chromatographed on Merck silica gel 60 to give title amide.

F. [lS-(lα,2α,3α,4α)]-2-[[3-[4-[[(4-Cyclohexylbutyl)amino]carbonyl]-lH-imidazol-2-yl]-7-oxabicyclo[2.2.l]hept-2-yl]methyl]-α,α-dimethylbenzenepropanoic acid, methyl ester

To a stirred mixture of Part E amide (0.94 mmol) in of dry $CH_2Cl_2$ at 0°C is added trifluoroacetic acid (TFA). The mixture is stirred at room temperature for 3 hours. The mixture is diluted with 40 mL of toluene and concentrated in vacuo. The crude imidazole-TFA salt is diluted with EtOAc and washed once with saturated $NaHCO_3$ solution. The aqueous layer is extracted with EtOAc. The combined EtOAc extracts are dried ($MgSO_4$), filtered and concentrated in vacuo. To this crude imidazoline in $CHCl_3$ is added $MnO_2$ (6.55 mmol). The mixture is stirred at room temperature for 64 hours at which time $MnO_2$ (6.55 mmol) is added. The mixture is stirred at room temperature for l day and another amount of $MnO_2$ (3.28 mmol) is added. The mixture is stirred at room temperature for one more day and again $MnO_2$ (2.l8 mmol) is added. The mixture is stirred at room temperature for l day and $MnO_2$ is filtered off through a pad of Celite and the pad is rinsed with $CHCl_3$ . The filtrate is concentrated in vacuo and chromatographed on Merck silica gel 60 to give title imidazole.

Example 6

[lS-(lα,2α,3α,4α)]-2-[[3-[4-[[(4-Cyclohexylbutyl)amino]carbonyl]-lH-imidazol-2-yl]-7-oxabicyclo[2.2.l]hept-2-yl]-methyl]-α,α-dimethylbenzenepropanoic acid, hydrochloride salt

To a stirred mixture of Example 5 imidazole (0.05 mmol) in l mL of methanol is added 2N KOH solution. The mixture is stirred at room temperature for 4 hours and concentrated in vacuo to remove methanol. The residue is diluted with $CH_2Cl_2$ and acidified to pH 2 by the addition of lN HCl solution. The aqueous layer is separated and extracted with $CH_2Cl_2$. The combined $CH_2Cl_2$ extracts are dried ($Na_2SO_4$), filtered and concentrated in vacuo. The crude product is dissolved in $CH_2Cl_2$ and combined with 4N HCl in ether. The resulting mixture is concentrated in vacuo and triturated in hot EtQAc. The mixture is cooled to room temperature and the solid formed is collected by filtration to give title hydrochloride salt.

Example 7

[1S-[1α,2α(Z),3α,4α]]-6-[3-[4-[[(4-Cyclohexylbutyl)amino]carbonyl]-2-oxazolyl]-7-oxabicyclo[2.2.1]hept-2-yl]-4-α,α-dimethylhexenoic acid

A. [(1,1-Dimethylethoxy)carbonyl]-N-(4-cyclohexylbutyl)-L-serinamide

To a solution of 575 mg of 4-cyclohexylbutylamine hydrochloride (3.0 mmol), 615 mg t-butyloxycarbonyl-L-serine (3.0 mmol, 1.0 equiv), 405 mg 1-hydroxybenzotriazole hydrate (3.0 mmol, 1.0 equiv), and 387 mg diisopropylethylamine (3.0 mmol, 1.0 equiv) in 10 mL dry tetrahydrofuran (THF) stirring under argon at 0°, was added 618 mg 1,3-dicyclohexylcarbodiimide (3.0 mmol, 1.0 equiv) in a single

portion. A precipitate slowly formed. After 1 hour the mixture was warmed to room temperature and stirred for 4 hours. After dilution with ethyl acetate, the mixture was filtered, and the filtrate was washed with a pH 1 salt solution (formed by mixing water, brine, and 1 M aqueous HCl solution). Further washing (twice) with 1 M $NaHCO_3$ was followed by drying over $Na_2SO_4$ and evaporation to give 1.1 g of crude title amide.

B. N-(4-Cyclohexylbutyl)-L-serinamide

To a solution of 1.1 g crude Part A amide in 4 mL $CH_2Cl_2$ at room temperature was added 4 mL trifluoroacetic acid. The mixture was stirred for 4 hours. After solvent evaporation, residual trifluoroacetic acid was azeotropically removed by rotoevaporation with $CHCl_3$. Flash chromatography (150 g silica, 10% [10% concentrated aqueous $NH_3$in $CH_3OH$] in $CH_2Cl_2$) gave, after azeotroping with toluene and exposure to high vacuum, 495 mg of pure title amine as a white solid. The yield of title amine was 68% overall from 4-cyclohexylbutyl amine hydrochloride.

C. [1S-[1α,2α(Z),3α,4α]]-6-[3-(Hydroxymethyl)-7-oxabicyclo[2.2.1]hept-2-yl]-4-α,α-dimethylhexenoic acid, methyl ester

To a partial solution of [4aR-(4aα,5β,8β,8aβ)]-octahydro-5,8-epoxy-lH-2-benzopyran-3-ol (prepared as described in U.S. Patent No. 4,l43,054) (23 mmol) and α,α-dimethyl-3-carboxypropyltriphenylphosphonium bromide (37 mmol) in dry THF under argon at 3°C is added dropwise over l hour a solution of of potassium t-amylate (68 mmol of a l.8M toluene solution) with mechanical stirring. The reaction is then run at room temperature for 90 minutes. A 0°C ice bath is introduced and the reaction is quenched by the addition of glacial acetic acid, over 30 minutes. Solvents are removed in vacuo (azeotroped with toluene). Water and concentrated HCl are added (pH 2.6).

The mixture is extracted with ethyl acetate, dried (sodium sulfate) and concentrated in vacuo. The crude material is dissolved in methanolic HCl, stirred for 24 h and concentrated in vacuo. The crude material is purified by flash chromatography (Merck silica) to give title methyl ester.

D. [1S-[1α,2α(Z),3α,4α]]-6-[3-(Carboxy)-7-oxabicyclo[2.2.1]hept-2-yl]-4-α,α-dimethylhexenoic acid, methyl ester

To a solution of Part C alcohol (7.6 mmol) in acetone under argon at 0°, is added slowly Jones' Reagent (2.6 M in $Cr^{VI}$). The resulting mixture is stirred for 20 minutes before 2-propanol is added to quench excess reagent. Still at 0°, 3 M aqueous $NaHSO_3$ solution is added with stirring until all salts dissolved. Brine was added, and extraction with ethyl acetate followed. After drying the extracts over $Na_2SO_4$, solvent evaporation, and flash chromatography (silica) afforded title acid.

E. [1S-[1α,2α(Z),3α(R*),4α]]-6-[3-[[[2-[(4-Cyclohexylbutyl)amino]-1-(hydroxymethyl)-2-oxoethyl]amino]-carbonyl]-7-oxabicyclo[2.2.1]hept-2-yl]-4-α,α-dimethylhexenoic acid, methyl ester

To a solution of Part D acid in DMF cooled in an ice-bath is added l-hydroxybenzotriazole (2.4 mmol), triethylamine (2.4 mmol) and Part B amine (2.4 mmol) then after several minutes WSC (2.4 mmol). The reaction mixture is stirred at 0° for 2 h then at 25° for l6 h, partitioned between ethyl acetate and lM HCl solution. The organic layer is washed with lM HCl, lM NaOH, brine, dried (magnesium sulfate) and concentrated in vacuo. Purification by flash chromatography (Merck silica) afforded title amide.

F. [1S-[1α,2α(Z),3α(R*),4α]]-6-[3-[4-[[(4-Cyclohexylbutyl)amino]carbonyl]-4,5-dihydro-2-oxazolyl]-7-oxabicyclo[2.2.1]hept-2-yl]-α,α-dimethyl-4-hexenoic acid, methyl ester

To a solution of pure Part E hydroxybisamide (0.48 mmol) in dry acetonitrile under argon at room temperature, is added triphenylphosphine (0.72 mmol, 1.5 equiv), triethylamine (0.72 mmol, 1.5 equiv), and $CCl_4$ (0.58 mmol, 1.2 equiv), and the mixture is stirred at room temperature for l8 h. Solvent evaporation followed by flash chromatography (silica) affords the pure title oxazoline.

G. [1S-[1α,2α(Z),3α,4α]]-6-[3-[4-[[(4-Cyclohexylbutyl)amino]carbonyl]-2-oxazolyl]-7-oxabicyclo[2.2.l]hept-2-yl]-4-α,α-dimethylhexenoic acid, methyl ester

To a solution of pure Part F oxazoline (0.40 mmol) in $CH_2Cl_2$ is added 200 mg $NiO_2$, and the heterogenous mixture is stirred at room temperature. Over l day, five additional aliquots of the reagent are added until the reaction is complete. The mixture is diluted with ethyl acetate, and this is stirred with 3 M aqueous $NaHSO_3$ solution until the black color of the $NiO_2$ disappeared and most of the solids dissolved. Extraction with ethyl acetate is followed by drying over $Na_2SO_4$ and evaporation. Flash chromatography (silica) affords pure title oxazole.

H. [1S-[1α,2α(Z),3α,4α]]-6-[3-[4-[[(4-Cyclohexylbutyl)amino]carbonyl]-2-oxazolyl]-7-oxabicyclo[2.2.1]hept-2-yl]-4-α,α-dimethylhexenoic acid

To pure Part G oxazole (0.19 mmol) in $CH_3OH$ at room temperature, is added 1.0 M aqueous NaOH solution. After stirring the mixture for l.3 hours, 1 M aqueous HCl solution is added to lower the pH to 1. Extraction with ethyl acetate followed. The extracts are dried over $Na_2SO_4$, and solvent evaporation gives crude title acid. Flash chromatography (Merck silica) affords pure title acid.

## Example 8

[lS-(lα,2α,3α,4α)]-3-[4-[[(4-(Cyclohexylbutyl)amino]carbonyl]-2-oxazolyl]-7-oxabicyclo[2.2.l]heptane-2-α,α-dimethylhexanoic acid

A solution of Example 7 acid in ethyl acetate and acetic acid is degassed via a vacuum-fill cycle with argon. To this solution is added l0% Pd/C and the atmosphere is exchanged for hydrogen by two vacuum-fill cycles. A slight positive pressure is maintained through use of a hydrogen balloon. The mixture is stirred at room temperature for 22.5 hours, diluted with $CH_2Cl_2$ and filtered through a polycarbonate filter to remove the catalyst. The filtrate is concentrated *in vacuo* to afford pure title acid.

## Example 9

[lS-(lα,2α,3α,4α)]-2-[[3-[4-[(Heptylamino)carbonyl]-2-oxazolyl]-7-oxabicyclo[2.2.l]hept-2-yl]methyl]benzene-α,α-dimethylpropanoic acid, methyl ester

To a solution of acid prepared in Example l, Part L (0.52 mmol) in dry $CH_2Cl_2$ (distilled from $P_2O_5$) is added l small drop of DMF, followed by (0.63 mmol) of oxalyl chloride. The reaction is stirred until gas evolution ceased (about 30 minutes), then the mixture is concentrated *in vacuo* to give the crude acid chloride as a pale yellow solid.

To a solution of crude acid chloride in dry $CH_2Cl_2$ (distilled from $P_2O_5$), cooled to 0°, is added (0.83 mmol) triethylamine, followed by the dropwise addition of a solution (0.62 mmol) of heptylamine in $CH_2Cl_2$. The reaction is stirred at 0° for l.5 hours, then partitioned between ethyl acetate/lM HCl. The ethyl acetate layer is separated, dried ($MgSO_4$) and concentrated *in vacuo* to give a crude orange solid. The crude solid is flash chromatographed (Merck silica) to give title ester.

## Example 10

[lS-(lα,2α,3α,4α)]-2-[[3-[4-[(Heptylamino)carbonyl]-2-oxazolyl]-7-oxabicyclo[2.2.l]hept-2-yl]methyl]benzene-α,α-dimethylpropanoic acid

To a mixture of (0.37 mmol) of Example 9 ester in THF/water is added (0.75 mmol, Aldrich) lithium hydroxide monohydrate. The reaction is stirred vigorously at room temperature for 3 hours, then quenched by the addition of lM HCl. The mixture is partitioned between ethyl acetate/water. The ethyl acetate layer is separated, dried ($MgSO_4$) and concentrated *in vacuo* to give a crude white solid. The crude solid is recrystallized to give title acid.

Example ll

[1S-[1α,2α(Z),3α,4α]]-6-[3-[5-[[(4-Cyclohexylbutyl)amino]carbonyl]-1H-imidazol-2-yl]-7-oxabicyclo[2.2.1]hept-2-yl]-4-α,α-dimethylhexenoic acid, methyl ester

The title compound may be prepared employing the procedures set out in Example 5 except that the Example 7, Part D acid is employed in place of the Example l, Part H acid.

Example l2

[lS-[lα,2α(Z),3α,4α]]-6-[3-[4-[[(4-Cyclohexylbutyl)amino]carbonyl]-lH-imidazol-2-yl]-7-oxabicyclo[2.2.l]hept-2-yl]-4-α,α-dimethylhexenoic acid

To a solution of Example ll ester in methanol is added 2N KOH. The reaction is stirred at room temperature for 4 hours. The methanol is removed in vacuo. The residue is taken up in methylene chloride and brought to pH 2 with lN HCl. After shaking, the aqueous layer is further extracted with methylene chloride. The combined organic layers are dried ($Na_2SO_4$) and concentrated in vacuo. The residue is taken up in methylene chloride and ethereal HCl was added. The mixture is concentrated in vacuo. Trituration with of ethyl acetate yields a white solid which is collected by filtration and dried to yield title acid.

Examples of additional compounds in accordance with the present invention which may be prepared following the procedures outlined in the specification and working Examples include, but are not limited to the following.

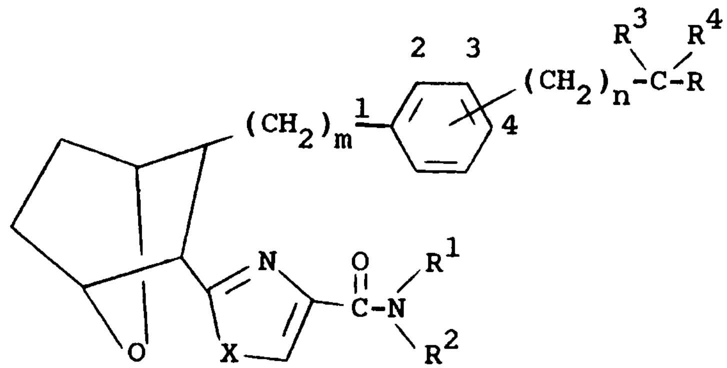

| Example No. | $(CH_2)_m$ m | $(CH_2)_n$ n | $-(CH_2)_n$ (position) | X | $R^1$ | $R^2$ | R | $R^3$ | $R^4$ |
|---|---|---|---|---|---|---|---|---|---|
| 13 | 1 | 2 | -(2) | O | $-C_6H_{13}$ | $CH_3$ | $CO_2H$ | $CH_3$ | $CH_3$ |
| 14 | 2 | 2 | -(2) | O | $-(CH_2)_4-$ 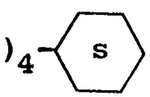 | $CH_3$ | $CO_2H$ | $C_2H_5$ | $C_2H_5$ |
| 15 | 2 | 3 | -(2) | O | $C_6H_5$ | $C_6H_5$ | $CO_2H$ | $CH_3$ | $CH_3$ |
| 16 | 1 | 2 | -(3) | NH | $-(CH_2)_3-$ 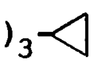 | H | $CO_2H$ | $CH_3$ | $CH_3$ |
| 17 | 2 | 2 | -(3) | O | 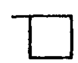 | $CH_2C_6H_5$ | $CO_2CH_3$ | $C_2H_5$ | $CH_3$ |

| Example No. | $(CH_2)_m$ m | $(CH_2)_n$ n | $-(CH_2)_n$ (position) | X | $R^1$ | $R^2$ | R | $R^3$ | $R^4$ |
|---|---|---|---|---|---|---|---|---|---|
| 18 | 1 | 2 | -(2) | O | 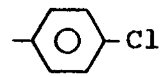 | H | $CO_2C_2H_5$ | $C_2H_5$ | $C_2H_5$ |
| 19 | 1 | 2 | -(2) | O | $(CH_2)_2C_6H_5$ | $CH_3$ | $CO_2H$ | $-CH_2CH_2-$ | |
| 20 | 1 | 2 | -(3.) | NH | $n-C_5H_{11}$ | H | $CO_2H$ | $C_2H_5$ | $CH_3$ |
| 21 | 1 | 2 | -(2) | O | $-(CH_2)_6-$ | | $CO_2H$ | $CH_3$ | $CH_3$ |
| 22 | 2 | 0 | -(3) | O | $n-C_4H_9$ | $n-C_4H_9$ | $CO_2H$ | $CH_3$ | $CH_3$ |
| 23 | 1 | 2 | -(2) | O | $-(CH_2)_4-$ 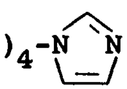 | H | $CO_2H$ | $C_4H_9$ | $C_4H_9$ |
| 24 | 2 | 2 | -(3) | O | $-(CH_2)_5-$ 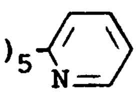 | H | $CO_2H$ | $CH_3$ | $C_2H_5$ |
| 25 | 1 | 2 | -(2) | O | $-(CH_2)_4-$ 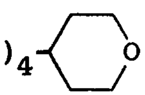 | H | $CO_2H$ | $CH_3$ | $CH_3$ |

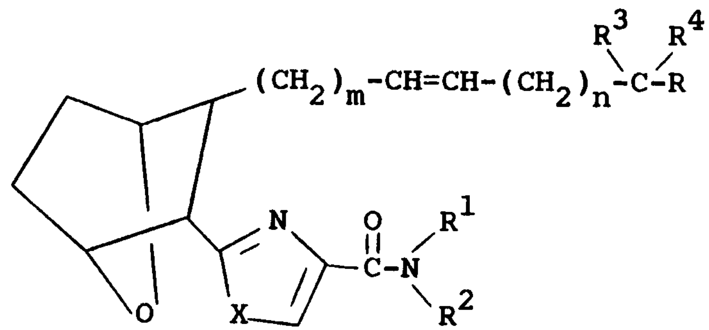

| Example No. | $(CH_2)_m$ m | $(CH_2)_n$ n | X | $R^1$ | $R^2$ | R | $R^3$ | $R^4$ |
|---|---|---|---|---|---|---|---|---|
| 26 | 1 | 2 | O | $C_6H_{13}$ | $CH_3$ | $CO_2H$ | $CH_3$ | $CH_3$ |
| 27 | 2 | 2 | O | $-(CH_2)_2-$ 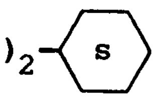 | $C_2H_5$ | $CO_2H$ | $C_2H_5$ | $C_2H_5$ |
| 28 | 2 | 3 | O | $C_6H_5$ | $C_6H_5$ | $CO_2H$ | $CH_3$ | $C_2H_5$ |
| 29 | 2 | 2 | O | 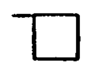 | $CH_2C_6H_5$ | $CO_2CH_3$ | $CH_3$ | $CH_3$ |

| Example No. | $(CH_2)_m$ m | $(CH_2)_n$ n | X | $R^1$ | $R^2$ | R | $R^3$ | $R^4$ |
|---|---|---|---|---|---|---|---|---|
| 30 | 1 | 2 | NH | $C_2H_5$ | H | $CO_2Li$ | $C_2H_5$ | $CH_3$ |
| 31 | 1 | 3 | NH | 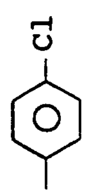 | $C_2H_5$ | $CO_2C_2H_5$ | $C_2H_5$ | $C_2H_5$ |
| 32 | 1 | 2 | O | $-(CH_2)_2C_6H_5$ | $CH_3$ | $CO_2H$ | $CH_3$ | $CH_3$ |
| 33 | 1 | 2 | O | $-(CH_2)_6-$ (for $R^1$ and $R^2$ together) | | $CO_2H$ | $CH_3$ | $CH_3$ |

## Claims

**1.** A compound having the formula

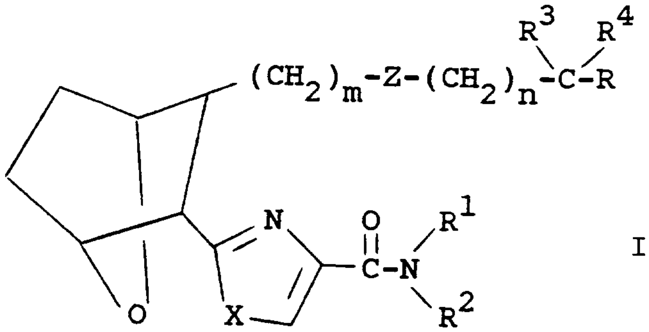

I

including all stereoisomers thereof, wherein

m is 1, 2 or 3;

n is 0, 1, 2, 3 or 4;

Z is $-(CH_2)_2-$, -CH = CH- or

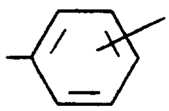

with the proviso that when Z is -CH = CH-, n is l, 2, 3 or 4;

R is $CO_2H$, $CO_2$alkali metal, or $CO_2$lower alkyl;

X is O or NH;

$R^1$ is hydrogen, lower alkyl, lower alkenyl, lower alkynyl, aryl, aralkyl, cycloalkyl, cycloalkylalkyl, cycloheteroalkyl, cycloheteroalkylalkyl, heteroaryl or heteroarylalkyl, or an amide

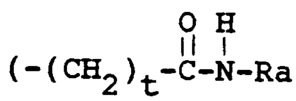 or 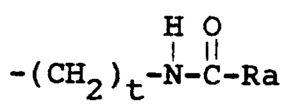

wherein t is l to l2 and Ra is lower alkyl, aryl, cycloalkyl or cycloalkylalkyl); each of $R^l$ being unsubstituted or optionally substituted with a lower alkyl, aryl, cycloalkyl or cycloalkylalkyl group;

$R^2$ is hydrogen, lower alkyl, aryl, or aralkyl, or

$R^1$ and $R^2$ together with the N to which they are linked form a 5- to 8- membered ring; and

$R^3$ and $R^4$ are the same or different and each is lower alkyl, $R^3$ and $R^4$ may be linked to form a 3- or 4-membered ring, including pharmaceutically acceptable salts thereof.

**2.** The compound as defined in Claim l having the formula

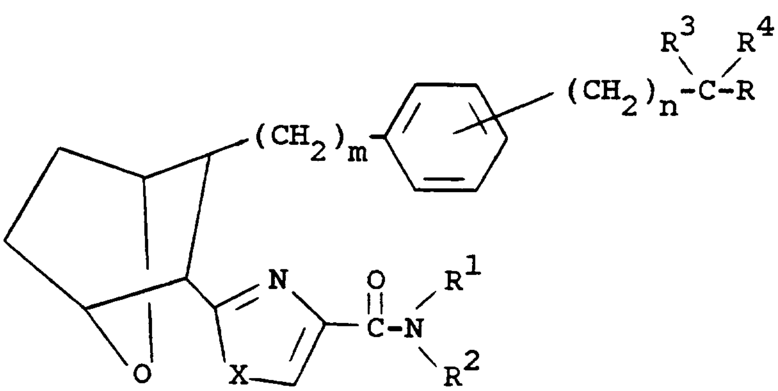

**3.** The compound as defined in Claim 2 having the formula

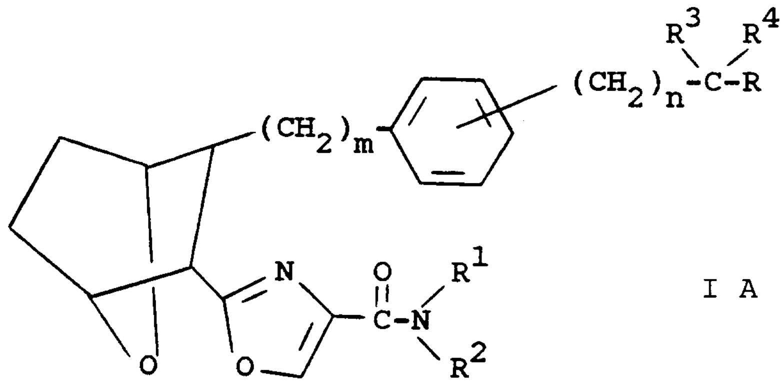

I A

4. The compound as defined in Claim 2 where m = 1 and n = 2.

5. The compound as defined in Claim 2 having the formula

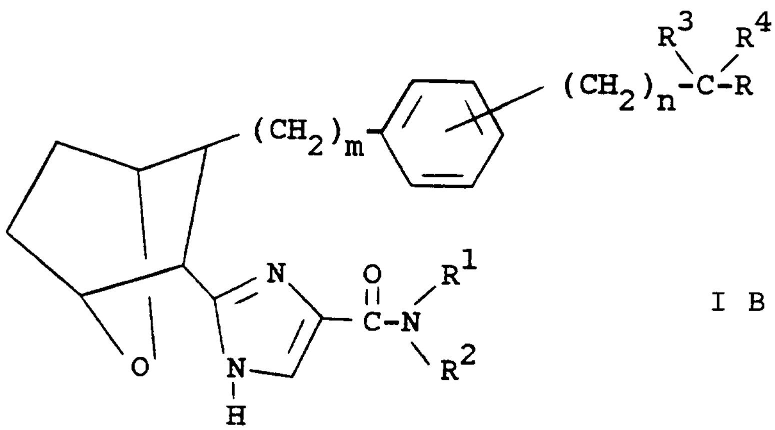

I B

6. The compound as defined in Claim 1 wherein $R^3$ and $R^4$ are each the same alkyl.

7. The compound as defined in Claim I wherein $R^3$ and $R^4$ are each methyl.

8. The compound as defined in Claim 3 having the formula

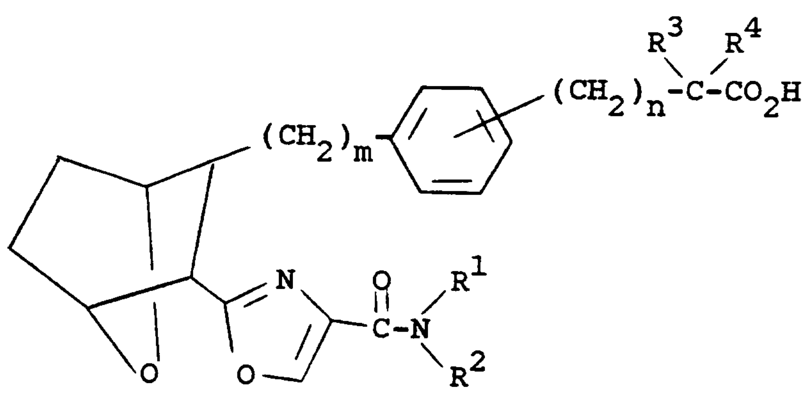

9. The compound as defined in Claim I wherein Z is

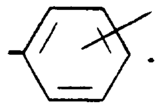

10. The compound as defined in Claim I wherein Z is $-(CH_2)_2-$ or -CH = CH-.

11. The compound as defined in Claim 2 having the name [IS-(Iα,2α,3α,4α)]-2-[[3-[4-[(pentylamino)-carbonyl]-2-oxazolyl]-7-oxabicyclo[2.2.I]hept-2-yl]methyl]-α,α-dimethylbenzenepropanoic acid, or esters or salts thereof; or [IS-(Iα,2α,3α,4α)]-2-[[3-[4-[[(4-cyclohexylbutyl)amino]carbonyl]-2-oxazolyl]-7-oxabicyclo[2.2.I]hept-2-yl]methyl]-α,α-dimethylbenzenepropanoic acid, or esters, or salts thereof.

12. The compound as defined in Claim 1 having the formula

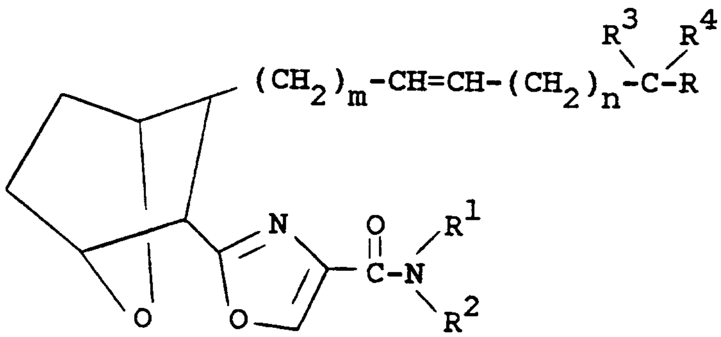

13. The compound as defined in Claim 12 where m = 1 and n = 2.

14. The compound as defined in Claim 12 having the formula

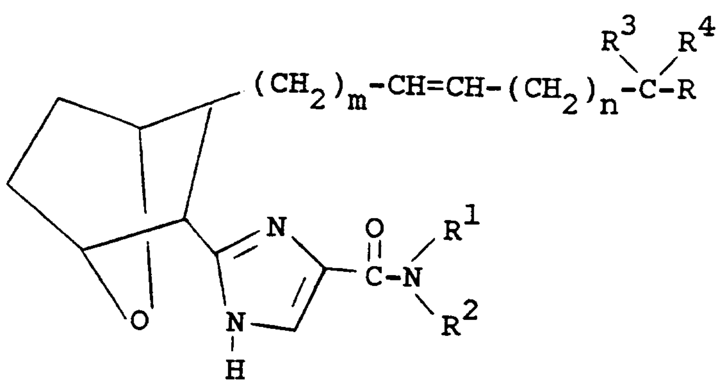

15. The compound as defined in Claim 12 wherein $R^3$ and $R^4$ are each the same lower alkyl.

16. The compound as defined in Claim 12 wherein $R^3$ and $R^4$ are each methyl.

17. A pharmaceutical composition comprising a compound as defined in anyone of claims 1 to 16.

18. Use of a compound according to anyone of claims 1 to 16 for the preparation of a pharmaceutical composition useful in inhibiting platelet aggregation and bronchoconstriction, whereby said pharmaceutical composition is such that it may be administered to the circulatory system.

**19.** Use as defined in Claim 18 wherein said pharmaceutical composition is such that the compound is administered in an amount within the range of from about 0.1 to about 100 mg/kg.

**20.** Use of a compound according to anyone of claims 1 to 16 for the preparation of a pharmaceutical composition useful in inhibiting platelet aggregation.

**21.** Use of a compound according to anyone of claims 1 to 16 for the preparation of a pharmaceutical composition useful in inhibiting bronchoconstriction associated with asthma.

**22.** Use of a compound according to anyone of claims 1 to 16 for the preparation of a pharmaceutical composition useful in improving post-ischemic myocardial function.

**23.** Use of a compound according to anyone of claims 1 to 16 for the preparation of a pharmaceutical composition useful in treating toxemia during pregnancy.

**24.** Use of a compound according to anyone of claims 1 to 16 for the preparation of a pharmaceutical composition useful in preventing or reducing venous thrombosis.

**25.** Use of a compound according to anyone of claims 1 to 16 for the preparation of a pharmaceutical composition useful in preventing or reducing platelet loss during extracorporeal circulation.

**26.** Use of a compound according to anyone of claims 1 to 16 for the preparation of a pharmaceutical composition useful in treating burn injuries and/or promoting wound healing, wherein the pharmaceutical composition is such that it may be used systemically or topically.

**27.** Use of a compound as defined in anyone of claims 1 to 16 and a thrombolytic agent for the preparation of a pharmaceutical composition useful in reducing post-ischemic myocardial injury within 6 hours of a myocardial infarction.

**28.** The use as defined in Claim 27 wherein said thrombolytic is t-PA, streptokinase, urokinase, prourokinase or anisoylated plasminogen-streptokinase activator complex.

**29.** A compound having the formula

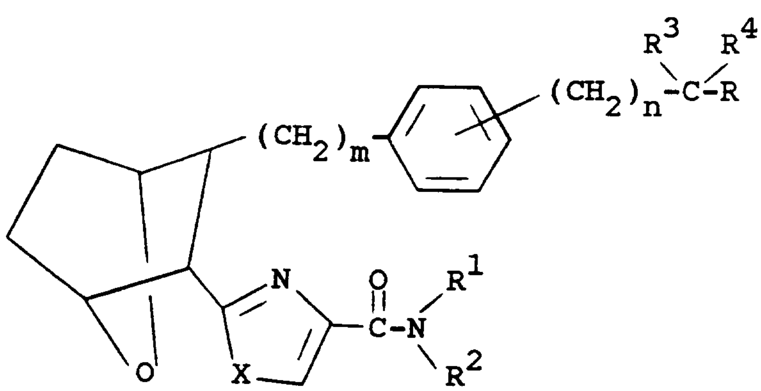

including all stereoisomers thereof, wherein

m is 1, 2 or 3;

n is 0, 1, 2, 3 or 4;

R is $CO_2H$, $CO_2$alkali metal or $CO_2$lower alkyl;

X is O or NH;

$R^1$ is lower alkyl, aryl, aralkyl, cycloalkyl, cycloalkylalkyl, saturated heterocycle, or aromatic heterocycle; and

$R^2$ is hydrogen, lower alkyl, aryl, or aralkyl, or $R^1$ and $R^2$ together with the N to which they are linked form a 5- to 8- membered ring; and $R^3$ and $R^4$ are the same or different and each is lower alkyl;

$R^3$ and $R^4$ may be linked to form a 3- or 4-membered ring, including pharmaceutically acceptable salts thereof.

**30.** A compound having the formula

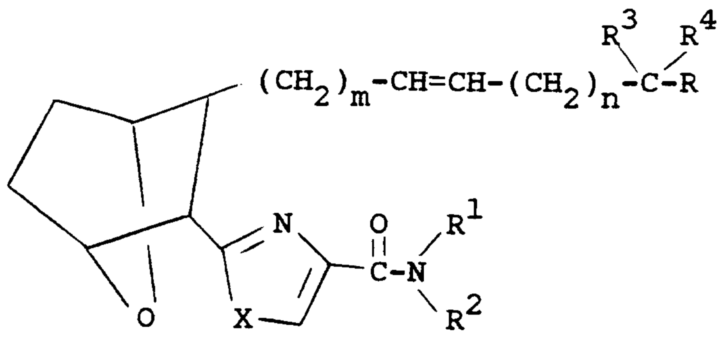

including all stereoisomers thereof, wherein

m is 1, 2 or 3;

n is 1, 2, 3 or 4;

R is $CO_2H$, $CO_2$alkali metal or $CO_2$lower alkyl;

X is O or NH;

wherein $R^I$ is lower alkyl, aryl, aralkyl, cycloalkyl, or cycloalkylalkyl;

$R^2$ is hydrogen, lower alkyl, aryl, or aralkyl, or $R^I$ and $R^2$ together with the N to which they are linked form a 5- to 8- membered ring; and $R^3$ and $R^4$ are the same or different and are each lower alkyl; $R^3$ and $R^4$ may be linked to form a 3- or 4-memebered ring, including pharmaceutically acceptable salts thereof.

European Patent Office

# EUROPEAN SEARCH REPORT

Application Number

EP 92 12 0196

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | EP-A-0 374 952 (E.R. SQUIBB & SONS, INC.)<br>* the whole document *<br>--- | 1-30 | C07D493/08<br>A61K31/41<br>A61K31/557<br>//(C07D493/08, 307:00,307:00) |
| Y | EP-A-0 391 652 (E.R. SQUIBB & SONS, INC.)<br>* the whole document *<br>--- | 1-30 | |
| Y | US-A-4 582 854 (S.E. HALL ET AL.)<br>* the whole document, particularly examples 65-69 *<br>----- | 1-30 | |
| | | | TECHNICAL FIELDS SEARCHED (Int. Cl.5 ) |
| | | | C07D |

The present search report has been drawn up for all claims

| Place of search | Date of completion of the search | Examiner |
|---|---|---|
| THE HAGUE | 26 FEBRUARY 1993 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)